# EUROPEAN PATENT APPLICATION

(11) **EP 4 342 890 A1**
(43) Date of publication of application: **27.03.2024**
(21) Application number: 22196879.5
(22) Date of filing: 21.09.2022
(51) Int. Cl.: C07D 401/14, C07D 411/14, C07D 498/18, A61P 35/00, A61K 31/4709

(54) **PLATFORM AND SCAFFOLD FOR FAP TARGETING AGENTS**

(71) Applicant: Erasmus University Rotterdam Medical Center, 3015 GE Rotterdam (NL)
(72) Inventor: SEIMBILLE, Yann, 3015 GE Rotterdam (NL); HOORENS, Mark Wilhelmus Henricus, 3015 GE Rotterdam (NL)
(74) Representative: V.O.

(57) **Abstract**

The present invention is directed to a scaffold and a compound for targeting fibroblast activation protein (FAP) in cancer-associated fibroblasts (CAFs). The scaffold comprises a (4-quinoinolyl)glycinyl-2-cyanopyrrolidine scaffold that is substituted on the 8^{th} position of the quinoinolyl moiety with a bridging carbon or oxygen atom according to formula (I) 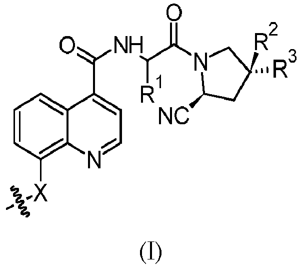 wherein X represents CH₂ or O; R¹ represents H, Me, CH(CH₃)C₂H₅, CH₂CH(CH₃)₂, CH(CH₃)₂, CH₂OH, CH₂SH, CH(OH)CH₃, CH₂C(O)NH₂, CH₂CH₂C(O)NH₂, (CH₂)ₘCO₂H, (CH₂)ₘNH₂, wherein m is 1-4; and R² and R³ each independently represent H or F.

## Description

The invention relates to compounds for targeting fibroblast activation protein (FAP) in cancer-associated fibroblasts (CAFs), in particular to FAP targeting agents based on a (4-quinoinolyl)glycinyl-2-cyanopyrrolidine scaffold.

Various FAP targeting agents based on the (4-quinoinolyl)glycinyl-2-cyanopyrrolidine scaffold have been developed and researched in the prior art to image and treat tumors (see Lindner et al. Cancers 2021, 13, 5744). Early FAP targeting agents are disclosed in Jansen et al. ACS Med. Chem. Lett. 2013, 4, 491-496 and in Jansen et al. J. Med. Chem. 2014, 57, 3053-3074, the latter describing fluorinated (4-quinolinoyl)-glycyl-2-cyanopyrrolidine scaffolds and UAMC-1110, a FAP inhibitor with nanomolar affinity to FAP.

For the FAP targeting agent to be clinically relevant, it must be functionalized with a payload such as a radioisotope, a fluorescent dye, a cytotoxic drug, or the like. To this end, the quinolinoyl moiety of the scaffold is generally used. The current reference compound in clinic is FAPI-46 (Loktev et al. Journal of Nuclear Medicine October 2019, 60 (10) 1421-1429). FAPI-46 was developed after the development of FAPI-04 (see Lindner et al. J. Nucl. Med. 2018; 59:1415-1422) and demonstrated improved tumor-to-organ ratios vis-à-vis FAPI-04.

Other functionalized FAP targeting compounds are described in WO2021/160825 and Millul et al. PNAS 2021 Vol. 118 No. 16 e2101852118 and referred to as OncoFAP. These compounds are functionalized on the quinoinolyl using a bridging nitrogen extended to a carboxylic acid group that allows connection to the payload. A drawback of this carboxylic acid group is that most metal chelating groups, prosthetic groups, optical dyes etc. are not readily available as carboxylic acid reactive. They are usually commercially available as amine reactive. Therefore, an additional linker (-NHCH₂-CH₂-NH-) is used in the OncoFAP compounds to expose an amine that can react with the payload.

Despite the recent advantages, there remains a need to provide an improved platform and improved FAP targeting agents based on the (4-quinoinolyl)glycinyl-2-cyanopyrrolidine scaffolds. Earlier research and development have however shown that FAP imposes strict structural requirements on its inhibitors and that steric or electronic parameters are not sufficient to rationalize the assay data (Jansen et al. J. Med. Chem. 2014, 57, 3053-3074). Developing new FAP targeting agent is accordingly challenging as it is hard if not impossible to predict which substituents and which position on the (4-quinoinolyl)glycinyl-2-cyanopyrrolidine scaffold is allowed to give good inhibitors.

An object of the present invention is to provide a platform for compounds and such compounds that show improved FAP binding affinity and/or tumor uptake, including tumor-to-organ distribution. Furthermore, an object of the present invention is to provide a platform that enables access to such compounds.

The present inventors have surprisingly found that substituting the 8^{th} position of the (4-quinoinolyl)glycinyl-2-cyanopyrrolidine scaffold with a carbon or oxygen atom gives entry into a variety of FAP targeting agents showing good FAP binding affinity and/or tumor uptake, including good tumor-to-organ distribution.

### Scaffold

The present invention is accordingly directed to a scaffold and a compound, in particular a pharmaceutical compound or a pharmaceutically acceptable salt thereof comprising a (4-quinoinolyl)glycinyl-2-cyanopyrrolidine scaffold that is substituted on the 8^{th} position of the quinoinolyl moiety with a bridging carbon or oxygen atom (herein also referred to as the scaffold). The bridging carbon or oxygen atom is connected or allows connection to a payload such as a therapeutic agent, a diagnostic agent, or a combination thereof, preferably to a radioisotope, a fluorescent dye, a drug or a combination thereof. The scaffold is depicted in the structure below, which also shows the numbering of the quinoinolyl moiety. 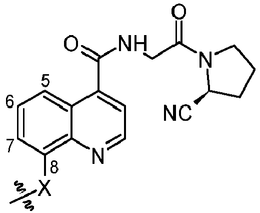 It may be appreciated that this scaffold may comprise further substituents such as fluorides and others, known for the FAP inhibitors based on the (4-quinoinolyl)glycinyl-2-cyanopyrrolidine scaffold .

More specifically, the present invention is directed to a compound, or a salt thereof, comprising a scaffold of formula (I) 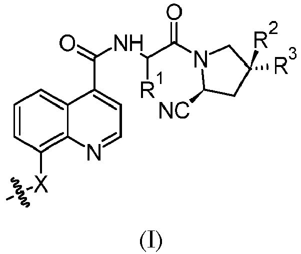 wherein
X represents CH₂ or O, preferably O;
R¹ represents H, Me, CH(CH₃)C₂H₅, CH₂CH(CH₃)₂, CH(CH₃)₂, CH₂OH, CH₂SH, CH(OH)CH₃, CH₂C(O)NH₂, CH₂CH₂C(O)NH₂, (CH₂)*_{q}*CO₂H, (CH₂)*_{q}*NH₂, wherein *q* is 1-4; and
R² and R³ each independently represent H or F.

In preferred embodiments, the moiety of formula (I) is conjugated to a payload such as a therapeutic agent, a diagnostic agent or a combination thereof via at least a spacer R⁴, as illustrated with formula (II) 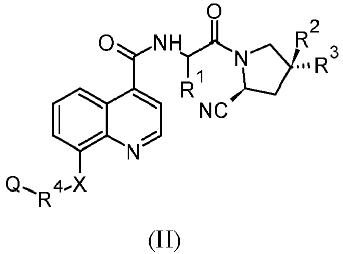 wherein
R⁴ represents a spacer, preferably an optionally substituted hydrocarbonic, preferably aliphatic, spacer; and
Q comprises the payload.

Of the (4-quinoinolyl)glycinyl-2-cyanopyrrolidine scaffold of the present invention, preferably at least one of R² and R³ is F, more preferably at least R² is F (such that the atom to which R² and R³ are connected has the *(S)* configuration or is achiral), most preferably R² and R³ are both F.

The R¹ substituent is preferably H.

### Spacer and linkers

The spacer represented by R⁴ preferably comprises a nitrogen atom, as illustrated in formula (IIIa), wherein R^{4'} represents an optionally substituted hydrocarbylene or an optionally substituted alkylene ether, preferably an alkylene, more preferably C₁-C₈ alkylene, that can be used to connect Q to the (4-quinoinolyl) glycinyl- 2-cyanopyrrolidine scaffold.

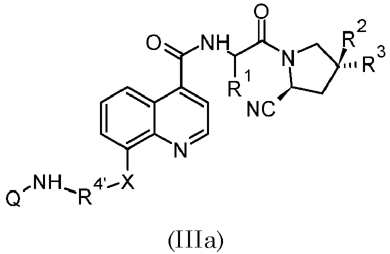

The spacer (R⁴ and R^{4'}) can be substituted or interrupted with one or more heteroatoms such as one or more of OH, NH₂, SO₂, and halogens such as F, Cl, Br and I. The spacer represented by R⁴ and R^{4'} is preferably based on an aliphatic C₁-C₈ amino terminated spacer. It was found that such a spacer leads to particularly good FAP binding affinities and tumor uptake. Accordingly, a particularly preferred embodiment of the compound of the present invention has a structure according to formula (IIIb) 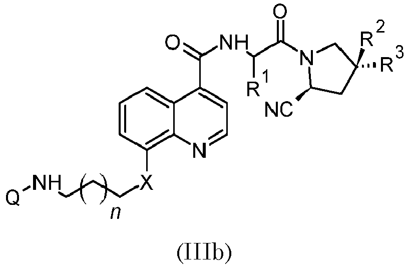 wherein *n* is 1-6, preferably 1.

The compound of structures according to formulae (IIIa) and (IIIb), including the Q functionality, can be readily prepared by the intermediate of formulae (VIIa) and (VIIb). 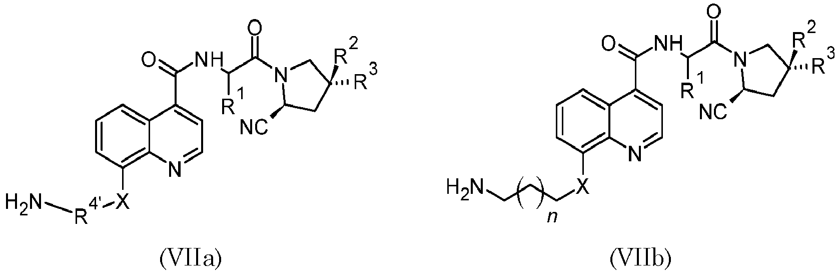 wherein X, R¹-R⁴, R^{4'} and *n* are as defined for formulae (IIIa) and (IIIb). Advantageously, this amine-terminated compound can be directly reacted with and/or linked to most commercially available metal chelating groups, prosthetic groups, optical dyes etc.. This intermediate compound is accordingly very versatile and an aspect of the present invention.

The group represented with Q (herein also simply referred to as Q) comprises at least one payload such as a therapeutic agent and/or diagnostic agent. It may be appreciated that in addition to this payload, Q may comprise linking functionalities (herein also referred to as linkers), one or more additional scaffolds and/or a combination of at least two of a radioisotope, a fluorescent dye and drug.

In a particular embodiment, Q contains a linker L that is connected to R⁴ or NHR^{4'}. The linker may serve to link one or more payloads and one or more scaffolds. The linker may assist in the solubility of the compound. The linker may be cleavable or non-cleavable (see for instance Kovtun et al. Cancer Letters 255 (2007) 232-240, Beck et al. Nature Reviews Drug Discovery 16 (2017) 315-337 and WO2021/160825) or a combination of both (*i*.*e*. one or more cleavable parts linking one or more payloads, and one or more other non-cleavable parts linking one or more other payloads or scaffolds).

The compound including linker L can be represented by formula (IV) 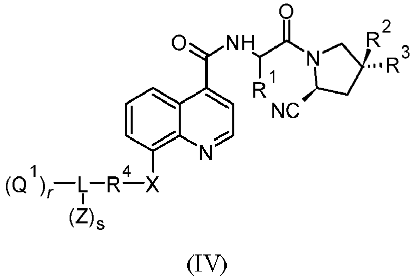 wherein L represents the linker, Q¹ comprises the payload, Z comprises an additional scaffold, *r* is 1 or more and s is 0 or more. It may be appreciated that the additional scaffold preferably has the structure according to formula (Z) 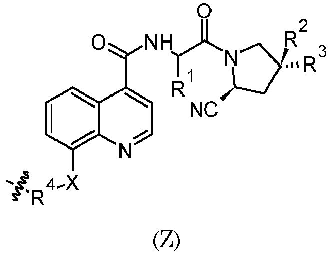 wherein X and R¹-R⁴ represent the groups as defined for formula (II).

The linker may comprise cleavable and non-cleavable sections, and combinations thereof. With a section is herein meant a structural feature linking the scaffold to the one or more payloads and optional addition scaffolds. Since the linker can link the scaffold to multiple payloads and additional scaffolds, the linker can contain multiple sections, each of which may be cleavable or non-cleavable. Whether a cleavable or non-cleavable section for the linker is preferred, depends *i.a.* on the payload. It may be appreciated that when the compound comprises a single payload (*i*.*e*. *r* = 1) and no additional scaffold (*i*.*e*. s = 0), the linker may comprises a single linker section.

Typically, when the linker is connecting a fluorophore dye and/or a radioisotope and stability of the scaffold and the payload is desired, the linker comprises a non-cleavable linker section. A non-cleavable linker section is also preferred for linking additional scaffolds.

Suitable non-cleavable linkers sections in this respect may be based on or comprise on linear or branched amino acids such as glycine, alanine, β-alanine, 3-aminopropionic acid, 4-aminobutyric acid, 5-aminovaleric acid, 6-aminohexanoic acid, 7-aminoheptanoic acid, 8-aminooctanoic acid, 9-aminononanoic acid, 10-aminodecanoic acid, 2-aminooctanoic acid, and the like. In some embodiments, the linker is a peptide spacer (Xaa)₁₋₄, wherein each Xaa is independently a proteinogenic or non-proteinogenic amino acid residue. Herein, each peptide backbone amino group may be independently optionally methylated. In particular embodiments, each non-proteinogenic amino acid residue is independently selected from the group consisting of a D-amino acid of a proteinogenic amino acid, *N^{ε}*,*N^{ε}*,*N^{ε}*-trimethyl-lysine, 2,3-diaminopropionic acid (Dap), 2,4-diaminobutyric acid (Dab), ornithine (Orn), homoarginine (hArg), 2-amino-4-guanidinobutyric acid (Agb), 2-amino-3-guanidinopropionic acid (Agp), β-alanine, 4-aminobutyric acid, 5-aminovaleric acid, 6-aminohexanoic acid, 7-aminoheptanoic acid, 8-aminooctanoic acid, 9-aminononanoic acid, 10-aminodecanoic acid, 2-aminooctanoic acid, 2-aminoadipic acid ( 2-Aad), 3-aminoadipic acid (3-Aad), 4-(aminomethyl)cyclohexane-1-carbonyl (Amcha), 4-amino- 1-carboxymethyl-piperidinyl (Pip), cysteic acid, diglycolic acid, and NH₂(CH₂)₂[O(CH₂)₂]*ₜ*(C(O)OH (wherein *t* = 1-36); and .

In a further preferred embodiment, the linker L comprise a moiety having a structure as shown in any of formulae La-Ln; 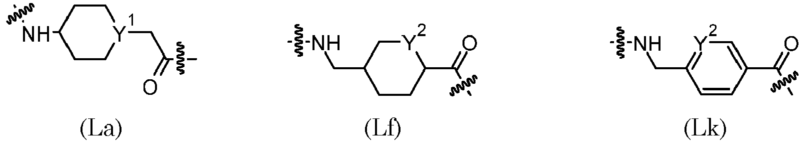 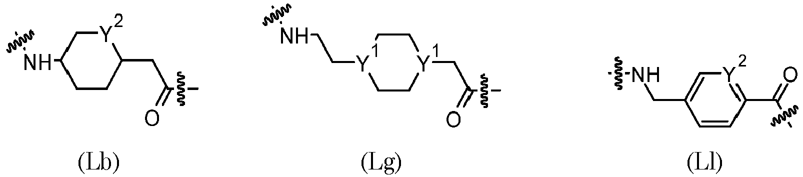 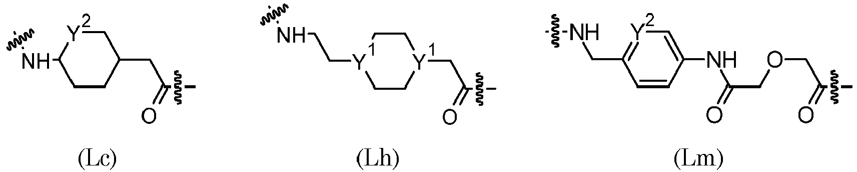 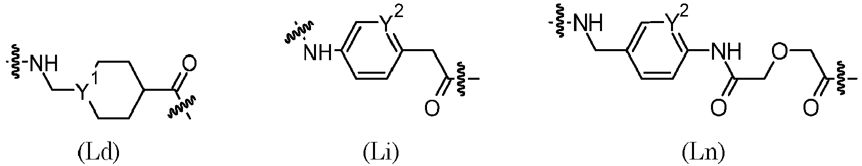 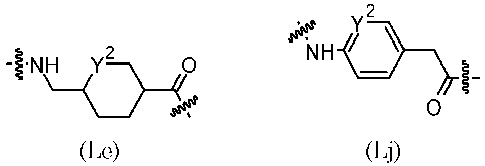 and wherein further
Y¹ is selected from the group consisting of C and N, preferably N for formula La, Lg and Lh and C for formula Ld; and
Y² is selected from the group consisting of C, N, and O, preferably N and C; more preferably C.

In a yet further preferred embodiment, the linker L comprises a moiety having a structure according to any of formulae Laa, Lfa, Lga, Lha, and Lma, which are specific versions of formulae La, Lf, Lg, Lh, and Lm respectively.

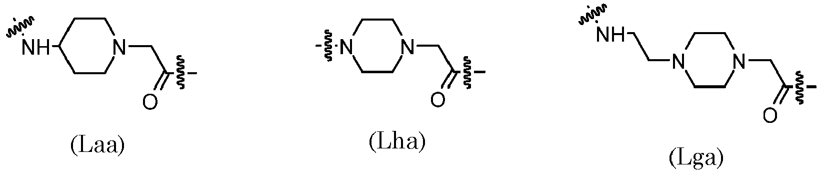

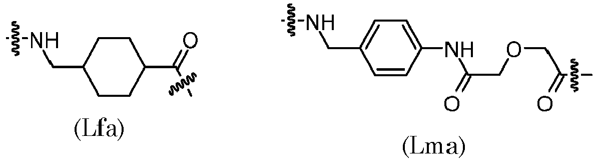

It may be appreciated that unless the stereochemistry of an atom has been specifically indicated herein, the stereochemistry of said atom is undefined which indicated that said structure represents all possible stereoisomers for said atom. For instance, structure Lfa represents at least two diastereoisomers: the *trans* and *cis*-isomers. However, preferably, the linker represented by this formula has the trans-configuration, as indicated by formula Lfa' below.

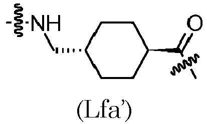

The linkers having the structures according to formulae IIaa and Lfa are based on 4-amino-1-carboxymethyl-piperidinyl (Pip) and 4-(aminomethyl)cyclohexane-1-carbonyl (Amcha), respectively. The Pip-containing linker, like any other amine-group containing linker disclose herein, may be cationic under physiological conditions, and as such could affect the global charge of the conjugate. Preferably, the linker comprises Amcha or Pip.

In particular embodiments, the linker may comprise a cleavable linker section comprising an *in vivo* cleavable moiety. Such as linker section is particularly preferred when the payload comprises a drug that benefits from delivery and release from the scaffold on a target location, e.g. near or in a tumor cell (see for instance Kovtun et al. Cancer Letters 255 (2007) 232-240, Beck et al. Nature Reviews Drug Discovery 16 (2017) 315-337, and WO2021/160825).

Enzyme-sensitive cleavable linkers sections are accordingly preferred linker sections and most preferred are cleavable sections that are sensitive to enzymes that are overexpressed in tumors, *e*.*g*. glutathione.

Examples of cleavable moieties that can be comprised in the cleavable linker section include amides, ester, carbamates, hydrazones, thiazolidine, methylene alkoxy carbamate and disulfides. The cleavable moiety may comprise one or more of such moieties and for example may include a peptide, oligosaccharide or another oligomeric sequence that can selectively be cleaved *in vivo.*

In preferred embodiments, the cleavable moiety comprises a disulfide moiety or a terminal thiol that enables disulfide linkage to the payload that comprises a terminal thiol as well (*e.g.* mertansine or DM1).

Accordingly, in particular preferred embodiment, the cleavable linker section is based on or comprises linear or branched mercapto carboxylic acids such as thioglycolic acid, thiolactic acid (also known as 2-mercaptopropionic acid), 3-mercaptopropionic acid (3-MPA) and the like. As such,the linker may comprise a moiety having the structure according to formula Lo 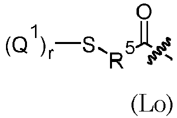 wherein R⁵ represents an optionally present second spacer, preferably an optionally substituted second aliphatic spacer, more preferably a C₁-C₆ alkylene, most preferably an ethylene.

The cleavable linker section may comprise a self-immolative moiety, which by a cascade of reactions is capable of releasing the payload unfunctionalized after activation (see for instance R.V. Gonzaga et al. Journal of Pharmaceutical Sciences 109 (2020) 3262-3281). Examples of suitable self-immolative moieties include *p*-amino benzyl carbamate either coupled to a valine-citrulline (Val-Cit-PAB) or β-glucuronide and those based on the Grob fragmentation (see for instance Ferhati et al. Org. Lett. 2021, 23, 21, 8580-8584).

The linker may be a dimeric linker that is linking the scaffold to one payload (*i*.*e*. *r* = 1 and s = 0 in formula (IV)). In other embodiments, the linker is multimeric, meaning that it is linking the scaffold to the payload and at least one additional payload and/or scaffold (*i*.*e*. *r* ≥ 1 and s + *r* ≥ 2 in formula (IV)). Thus, in particular embodiments, the linker links one or more additional scaffolds and/or a combination of at least two of a radioisotope, a fluorescent dye and drug to the scaffold. Generally, when the linker is linking more than 5 entities, the compound is too big and renal excretion is unfavorably hindered. Hence, the linker typically links the scaffold to the payload and to maximum three additional payloads and/or scaffolds (*i*.*e*. s + *r* = 1, 2, 3 or 4 while *r* ≥ 1 in formula (IV)).

Depending on the payload, appropriate linkers can be selected. For instance, if two payloads or one payload and one additional scaffold, of which one has a free carboxylic acid and another one has a free amine are to be linked, the linker can be based on glutamic acid. Similarly, if two payloads, each with a free carboxylic acid, are to be linked, the linker can be based or comprise a lysine or an analogue thereof. Accordingly, a particular embodiment is for example a compound having a structure according to formula (V) 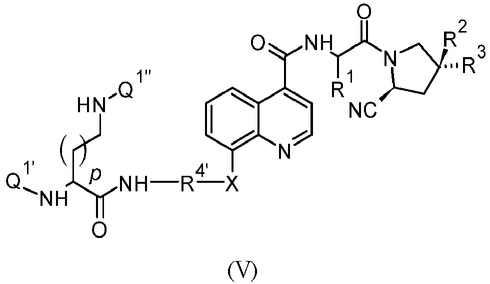 wherein p is 1-6, preferably 3; and
Q^{1'} and Q^{1"} each independently comprise a payload such as radioisotope, a fluorescent dye, and/or a drug.

In embodiments wherein the linker is linking the scaffold with one additional scaffold, the linker may comprise a multimeric moiety according to any of formulae (Lp) to (Lv) 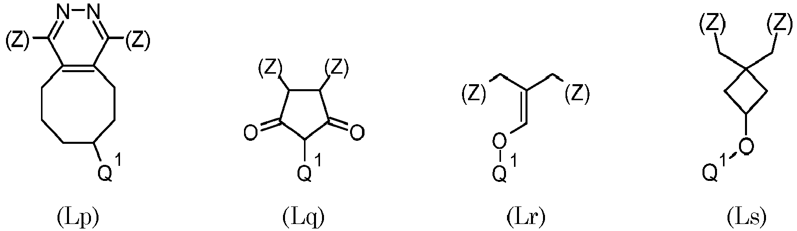 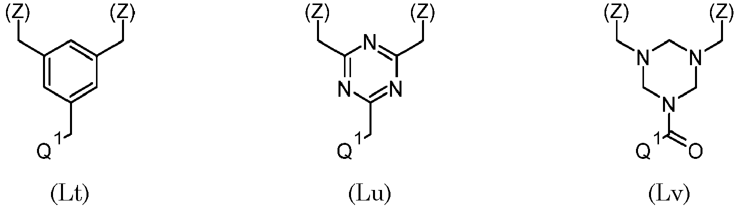 which formulae each show the position of the scaffolds (Z) and the payload Q¹ relative to each other with respect to the multimeric moiety. It may be appreciated the compound and the linker may comprise additional atoms and/or linking sections between the multimeric moiety and (Z) and Q¹ shown in formulae (Lp)-(Lv). Scaffolds (Z) are structurally the scaffold as described herein above. The scaffolds are preferably structural the same, but may be different in terms *of e.g.* X and R¹-R⁴.

### Diagnostic and therapeutic agents

The present invention is not particularly limited to specific payloads, diagnostic and therapeutic agents. Advantageously, the present invention allows a large variety of payloads to be used in combination with the scaffold, without compromising the FAP affinity and/or tumor uptake of the compound. It may be appreciated that appropriate payloads and agents can be selected based on the desired use such as diagnosis and treatment.

The payloads that are known for previously developed FAP targeting agents as for instance disclosed in WO2019/154859 and WO2021/160825 can also suitably be used with the present invention.

### Chelators and radioisotopes

In a particular embodiment, the payload comprises a radioisotope complexed to a chelator. The radioisotope may be any suitable radioisotope. See for instance Tornesello et al., Molecules 2017, 22(8), 1282, US 2021/0402016A1, WO2021/005125A1 and Price and Orvig, Chem. Soc. Rev., 2014, 43, 260-290 and references cited therein. The radioisotope may be a α- or β-particle emitter, an Auger emitter, a positron emitter and/or a γ-emitter. The α-particle emitters and β-particle emitters, such as ⁹⁰Y, ²¹²Pb, ¹⁷⁷Lu, ¹⁸⁸Re, ¹⁸⁶Re, ⁶⁷Cu, ⁶⁴Cu, ^{195m}Pt, ²¹²Bi, ²¹³Bi, ²¹¹At, ²²⁵Ac, ¹³¹I and the like can be used for therapy. Suitable γ-emitters such as ^{99m}Tc, ⁶⁷Ga, ¹¹¹In and the like can be used for SPECT imaging, while positron emitters such as ⁶⁸Ga, ⁶⁴Cu,¹⁸F and the like are useful for PET imaging. Accordingly, the radioisotope M for the present invention is preferably is selected from the group consisting of ¹⁸F, ¹²³I, ¹²⁴I, ¹²⁵I, ¹³¹I, ⁷⁶Br, ⁷⁷Br, ²¹²Pb, ²⁰³Pb, ⁶⁴Cu, ⁶⁷Cu ²¹²Bi, ⁶⁸Ga, ²¹³Bi, ²²⁵Ac, ²⁴³Am, ²¹¹At, ²¹⁷At, ¹⁵⁴Dy, ¹⁴⁸Gd, ¹⁴⁶Sm, ¹⁴⁷Sm, ¹⁴⁹Tb, ¹⁵²Tb, ¹⁵⁵Tb, ¹⁶¹Tb, ¹⁶⁵Er, ⁷²As, ⁷⁷As, ⁴⁷Sc, ¹⁸⁸Re, ¹⁸⁶Re, ¹⁰⁵Rh, ¹⁰⁹Pd, ¹⁹⁹Au, ¹⁷⁵Yb, ¹⁴²Pr, ^{114*m*}In, ^{94*m*}Tc, ^{99*m*}Tc, ²²⁷Th, ²²⁹Th, ⁵⁹Fe, ⁶⁰Cu, ⁶¹Cu, ⁶²Cu, ⁶⁷Ga, ⁴⁴Sc, ⁸⁹Zr, ⁹⁰Nb, ⁸⁶Y, ⁹⁰Y, ¹¹¹In, ¹⁷⁷Lu, ^{117*m*}Sn, ¹⁵³Gd, ¹⁵³Sm, and ¹⁶⁶Ho, preferably from the group consisting of ¹⁸F, ⁶⁴Cu, ⁶⁷Ga, ⁶⁸Ga, ⁹⁰Y, ¹¹¹In, ¹⁷⁷Lu, ²¹²Pb and ²²⁵Ac, most preferably from the group consisting of ¹⁸F, ⁶⁸Ga, ¹¹¹In, ²²⁵Ac and ¹⁷⁷Lu.

In the field, various chelators are known to bind radioisotopes. The chelator for the present invention is a pharmaceutically acceptable chelator. The chelator is typically selected based on the radioisotope that is desired for the diagnosis or treatment as not all chelators are equally suitable for all radioisotopes. Typical the radioisotope chelator comprises a cyclic or branched polyaminopolycarboxylic moiety or amide derivative thereof. See for instance Tornesello et al., Molecules 2017, 22(8), 1282, US2021/0402016A1, WO2021/005125A1 and Price and Orvig, Chem. Soc. Rev., 2014, 43, 260-290 and references cited therein.

In preferred embodiments of the present invention, the radioisotope chelator is based on compounds selected from the group consisting of
- DOTA (1,4,7,10-tetraazacyclodocecane-N,N',N",N"'-tetraacetic acid, also known as tetraxetan) and derivatives such as p-SCN-Bn-DOTA (2-(4-isothiocyanatobenzyl)-1,4,7,10-tetraazacyclododecane tetraacetic acid);
- PSC (1,4,7,10-tetraazacyclododecane-7-acetamide-1,4,10-triacetic acid);
- DO3A (1,4,7,10-tetraazacyclodocecane-N,N',N"-triacetic acid);
- DOTAGA (1,4,7,10-tetraazacyclododececane, 1-(glutaric acid)-4,7,10-triacetic acid);
- DO3AM (2,2',2"-(1,4,7,10-tetraazacyclododecane-1,4,7-triyl)triacetamide);
- DOTAM (2-[4,7,10-tris(2-amino-2-oxoethyl)-1,4,7,10-tetrazacyclododec-1-yl]acetamide) and derivatives such as p-SCN-Bn-TCMC (2-(4-isothiocyanatobenzyl)-1,4,7,10-tetraaza-1,4,7,10-tetra(2-carbamoylmethyl)cyclododecane);
- NOTA (1,4,7-triazacyclononane-N,N',N"-triacetic acid);
- NODAGA (1-(1,3-carboxypropyl)-4,7-carboxymethyl-1,4,7-triazacyclononane);
- NODASA (1,4,7-triazacyclononane-1-succinic acid-4,7-diacetic acid);
- CB-DO2A (4,10-bis(carboxymethyl)-1,4,7,10-tetraazabicyclo[5.5.2]tetradecane);
- 3p-C-DEPA (2-[(carboxymethyl)][5-(4-nitrophenyl-1-[4,7,10-tris-(carboxymethyl)-1,4,7,10-tetraazacyclododecan-1-yl]pentan-2-yl)-amino] acetic acid);
- TCMC (1,4,7,10-tetrakis(carbamoylmethyl)1,4,7,10-tetraazacyclododecane);
- DTPA (diethylenetriaminepentaacetic acid) and DTPA derivatives such as CHX-A"-DTPA (2-(p-isothiocyanatobenzyl)cyclohexyldiethylenetriaminepentaacetic acid and 1B4M-DTPA;
- TETA (1,4,8,11-tetraazacyclotetradecane 1,4,8,11-tetraacetic acid) and analogues and derivatives such as C-NETA;
- NE3TA ((7-[2-[carboxymethyl)amino]ethyl]-1,4,7-triazacyclononane-1,4-diacetic acid) and derivatives such as C-NE3TA;
- CB-TE2A (4,11-bis(carboxymethyl)-1,4,8,11 tetraazabicyclo[6.6.2]hexadecane);
- NETA ({4-[2-(bis-carboxymethylamino)-ethyl]7-carboxymethyl-[1,4,7]triazonan-1-yl)-acetic acid); and NETA derivatives such as
   3p-C-NETA (see Sun et al. ACS Omega 2020, 5, 44, 28615-28620)
- H₂azapa (N,N'-[1-benzyl-1,2,3-triazole-4-yl]methyl-N,N'-[6-(carboxy)pyridin-2-yl)-1,2-diaminoethane) and other picolinic acid derivatives such as H₂dedpa (1,2-[6-(carboxy)-pyridin-2-yl)methylamino)ethane, H₄octapa (N,N'-bis(6-carboxy-2-pyridylmethyl)-
- ethylenediamine-N,N'-diacetic acid), H₄py4pa, H₄Pypa, H₆phospha, H₄CHXoctapa, H₅decapa (N,N"-[[6-(carboxy)pyridin-2-yl]methyl]-
- diethylenetriamine-N,N',N"-triacetic acid), and H₄neunpa-p-Bn-NO₂;
- SHBED (N,N'-bis(2-hydroxy-5-sulfobenzyl)ethylenediamine-N,N'-diacetic acid);
- HBED (N,N'-bis(2-hydroxybenzyl)-ethylenediamine-N,N'-diacetic acid);
- H2-MACROPA (N,N'-bis[(6-carboxy-2-pyridil)methyl]-4,13-diaza-18-crown-6);
- PCTA (3,6,9,15-tetraazabicyclo[ 9.3.1]pentadeca-1(15),11,13-triene-3,6,9-triacetic acid);
- Me-3,2-HOPO (see Ramdahl, Bioorganic & Medicinal Chemistry Letters 26 (2016) 17, 4318-4321);
- CB-TE1A1P (1,4,8,11-tetraazacyclotetradecane-l-(methanephosphonic acid)-8-(methanecarboxylic acid));
- CB-TE2P (1,4,8,11-tetraazacyclotetradecane-1,8-di(methanephosphonic acid);
- MM-TE2A (N-monomethyl 1,8-N,N'-bis-(carboxymethyl)-1,4,8,11-tetraazacyclotetradecane);
- DM-TE2A (N,N'-dimethyl 1,8-N,N'-bis-(carboxymethyl)-1,4,8,11-tetraazacyclotetradecane);
- sarcophagine and sarcophagine derivatives such as SarAr (1-N-(4-aminobenzyl)-3,6,10,13,16,19-hexaazabicyclo[6.6.6]-eicosane-1,8-diamine, diamSar, AmBaSar, and BaBaSar;
- TRAP (1,4,7-triazacyclononane-1,4,7-tris[methyl(2-carboxyethyl)phosphinic acid) and analogues such as NOPO (1,4,7-triazacyclononane-1,4-bis[methylene(hydroxymethyl)phosphinic acid]-7-[methylene(2-carboxyethyl)phosphinic acid]);
- AAZTA (1,4-bis(hydroxycarbonyl methyl)-6-[bis(hydroxylcarbonylmethyl)] amino-6-methyl perhydro-1,4-diazepine);
- DATA and DATA derivatives;
- CP256 (4-acetamido-N1,N7-bis-[(3-hydroxy-1,6-dimethyl-4-methylene-1,4-dihydropyridin-2-yl)methyl]-4-(3-[(3-hydroxy-1,6-dimethyl-4-methylene-1,4-dihydropyridin-2-yl)methylamino]-3-oxopropyl)heptanediami) and its derivative YM103 (4-(3-[3-(2,5-dioxo-2,5-dihydro-1H-pyrrol-1-yl)propanamido]propanamido)-N1,N7-bis[(3-hydroxy-1,6-dimethyl-4-methylene-1,4-dihydropyridin-2-yl)methyl]-4-(3-[(3-hydroxy-1,6-dimethyl-4-methylene-1,4-dihydropyridin-2-yl)methylamino]-3-oxopropyl)heptanediamide);
- PCTA (6,9,15-tetraazabicyclo[9.3.1]pentadeca-1(15),11,13-triene-3,6,9,-triacetic acid);
- BCPA (see Price and Orvig, Chem. Soc. Rev., 2014, 43, 260-290);
- DFO (desferrioxamine) and DFO derivatives ;
- a trithiol chelate;
- mercaptoacetyl;
- hydrazinonicotinamide;
- dimercaptosuccinic acid;
- 1,2-ethylenediylbisL-cysteine diethyl ester;
- methylenediphosphonate;
- hexamethylpropyleneamineoxime;
- hexakis(methoxy isobutyl isonitrile);
and analogues thereof.

Most preferred are the DOTA and NOTA chelators as these chelate particularly well to ¹⁸F, ⁶⁸Ga, ¹¹¹In, ¹⁷⁷Lu and ²²⁵Ac.

Preferably, the chelator is bound to the linker via one of the amide groups or one of the carboxylic acid groups of the chelator, leading to an amide bond. However, it may also be bound via one of its carbon atoms. For linkage to a carbon atom, the chelator may be appropriately equipped with an isothiocyanate functionality, for instance an isothiocyanatobenzyl. Examples of such equipped chelators include pSCN-Bn-DOTA and p-SCN-Bn-TCMC.

In preferred embodiments, the linker is bound to one of the carboxylic acid groups of the chelator.

Accordingly, the radioisotope complexed to the chelator in particular embodiments has a structure according to any of formula Qa-Qh, preferably Qa, Qg or Qh, more preferably Qa or Qg. 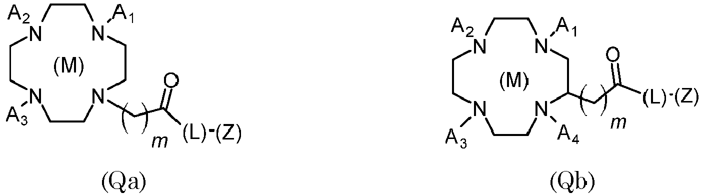 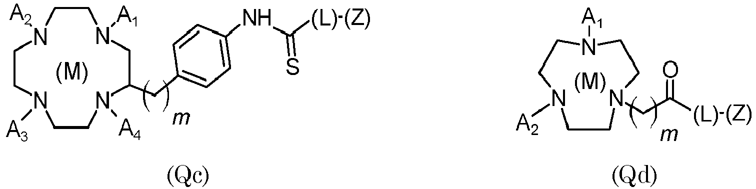 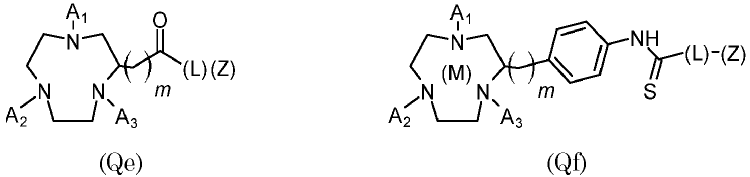 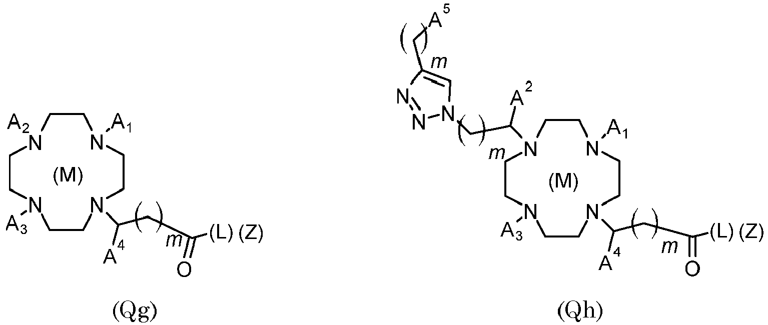 wherein m are individually 0-6, preferably 1;
A¹-A⁴ are independently selected from the group consisting of H, alkyls, aliphatic acids, such as carboxylic acids like CH₂CO₂H and CO₂H, and amides and esters thereof;
A⁵ represents a fluoride containing group or precursor therefor such as 1,4-butane sultone-1-yl (1,2λ⁶-oxathiane-2,2-dione-3-yl or 2,2-dioxido-1,2-oxathian-3-yl), CH(SO₃H)(CH₂)₃F and/or N(CH₃)₂)CH₂BF₃;
(M) represents the radioisotope;
(L) represents the optionally present linker or optionally present core as described herein; and
(Z) represents the scaffold as described herein.

Of the structures according to any of formula Qa-Qh, A₁-A₄ are preferably independently selected from the group consisting of H, (C₁-C₆)-alkyl, (C₁-C₆)-alkylene-(CO₂A⁶), (C₁-C₆)-alkylene-(C(O)NA⁶A⁷), wherein A⁶ and A⁷ are independently selected from the group consisting of H and (C₁-C₆)-alkyls, preferably H. Most preferably, A¹-A³ are CH₂CO₂H and A⁴ is CO₂H to aid water solubility of the compound. In a further preferred embodiment, A¹-A⁴ are all the same and selected from the group consisting of -CH₂CO₂H and -CH₂C(O)NH₂.

It may be appreciated that (L) may also be absent.

In particular embodiment, the chelator may be combined with a fluoride label, as illustrated in formula (Qh) wherein A⁵ represents CH(SO₃H)(CH₂)₃F and/or N(CH₃)₂)CH₂BF₃. This fluoride may be an ¹⁸F isotope, suitable in imaging such as PET imaging. The compound may also comprise a precursors for the preparation of such a compound, as illustrated in formula (Qh) wherein A⁵ represents 1,4-butane sultone (1,2λ⁶-oxathiane-2,2-dione).

### Fluorophores

The payload may be a dye, preferably a fluorescent dye such as a dye selected from the group consisting of cyanines, phtalocyanines,rhodamines, fluoresceins, xanthenes, coumarines, styryls, porphines, fluorescent organometallic complexes, oxanines, perylenes, acridines, boron-dipyrromethenes, and the like.

Particularly suitable fluorescent dyes include cyanines and/or phtalocyanines. Preferred dyes are CY5 dyes, in particular sulfo-CY5.

### Drugs

The payload may be a drug, preferably a cytotoxic or cytostatic drug. Examples thereof include some of the radioisotopes described herein-above, as well as molecular drugs. Suitable drugs are also those described in WO2021/160825, which is incorporated herein in its entirety. Particular suitable drugs include Hsp90 inhibitors such as geldanamycin analogues, radicicol analogues, Zelavespib (PU-H71), Onalespib (AT13387), SNX-0723, HSP990, YC-72-AB85, Luminespib (AUY922), VER-49009, and NMS-E973. Most preferably, the drug comprises Mertansine (DM1).

### Exemplary compounds

In a particularly preferred embodiment, the pharmaceutical compound, or a pharmaceutically acceptable salt thereof, has a structure according to any of formulae eFAP-6 to eFAP-20 below. Preferably, eFAP-6, eFAP-8, eFAP-9, eFAP-10, eFAP-11, eFAP-12, eFAP-13, eFAP-14, eFAP-15, eFAP-16, eFAP-19 and eFAP-20 comprise a radioisotope, more preferably a ¹¹¹In, ⁶⁸Ga, ¹⁷⁷Lu or ²²⁵Ac radioisotope. The compound eFAP-6 is particularly suitable for use in radioisotope medical imaging. The compounds eFAP-8 to eFAB-13 are particularly suitable for use in radioisotope-based tumor therapy, especially in view of their favorable tumor retention. The compound eFAB-17 is particularly suitable for use in image-guided surgery for the treatment of cancer. The compound eFAB-18 is particularly suitable for use in the treatment of tumors. The compound eFAB-19 is a biofunctionalized compound suitable for treatment of cancer by release of DM1 and radioisotope therapy with *e.g.* ¹⁷⁷Lu.

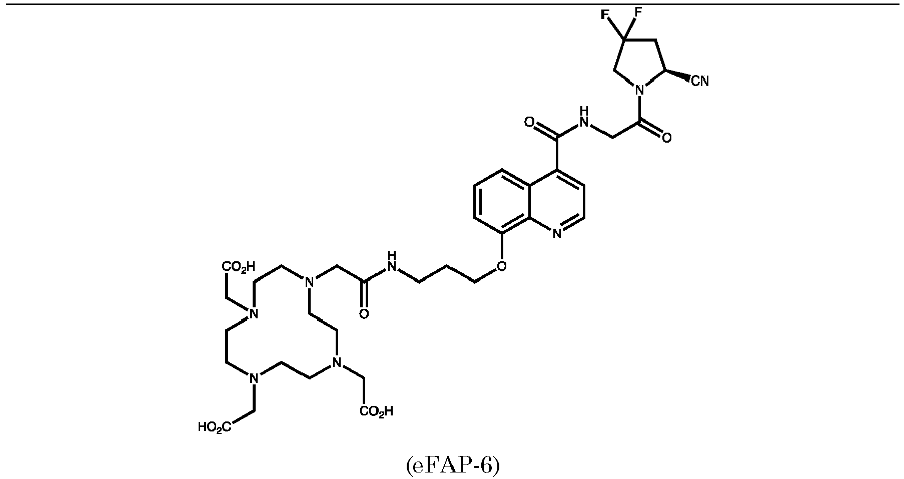

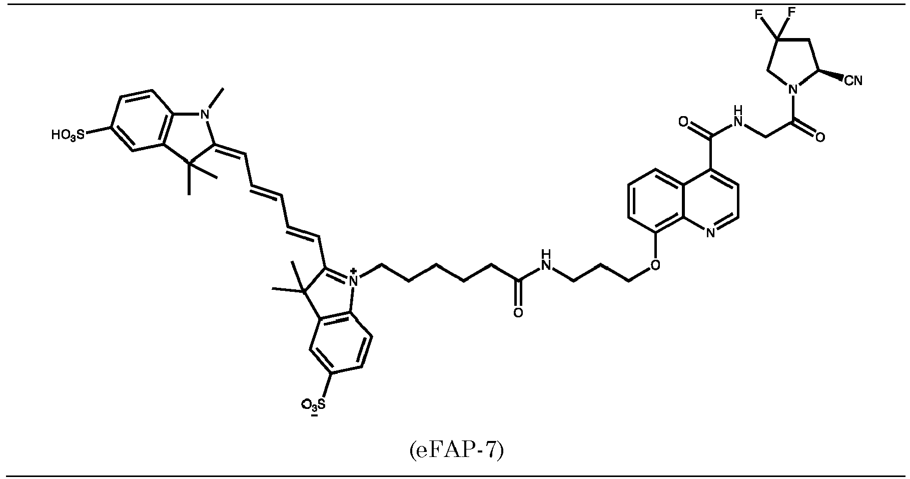

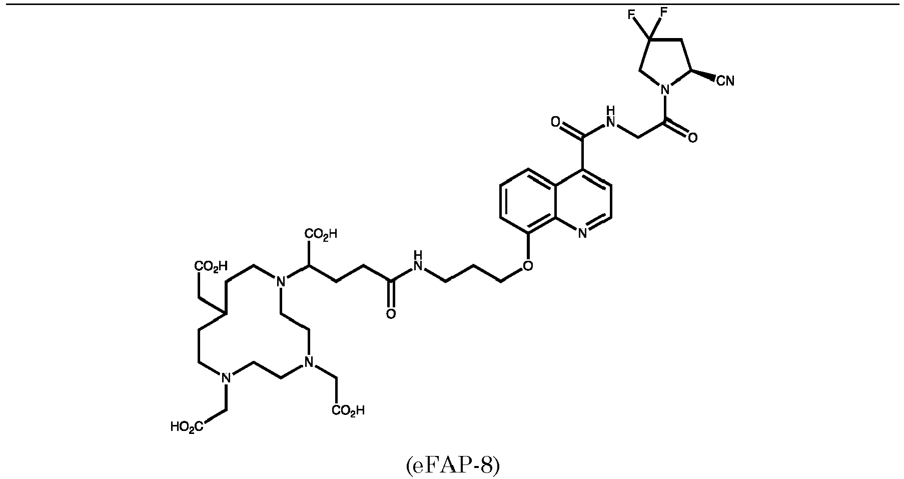

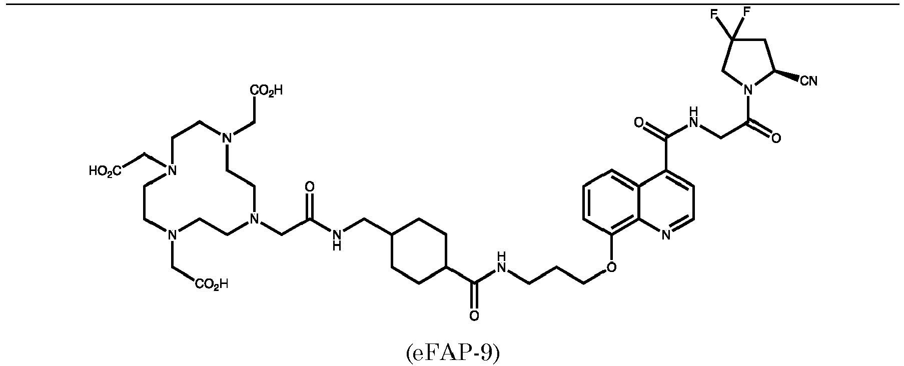

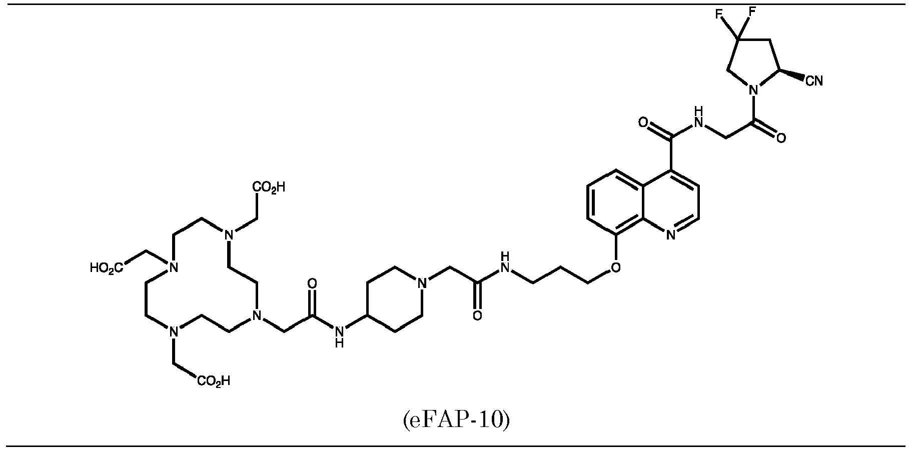

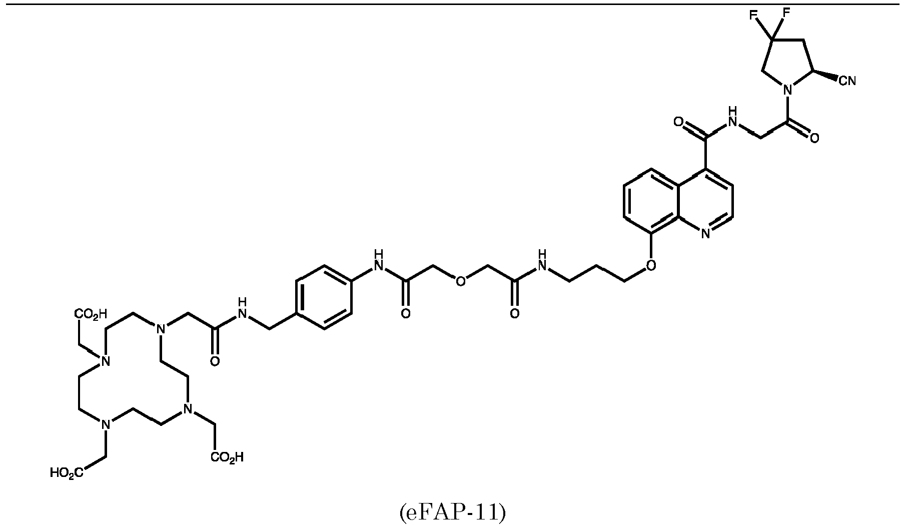

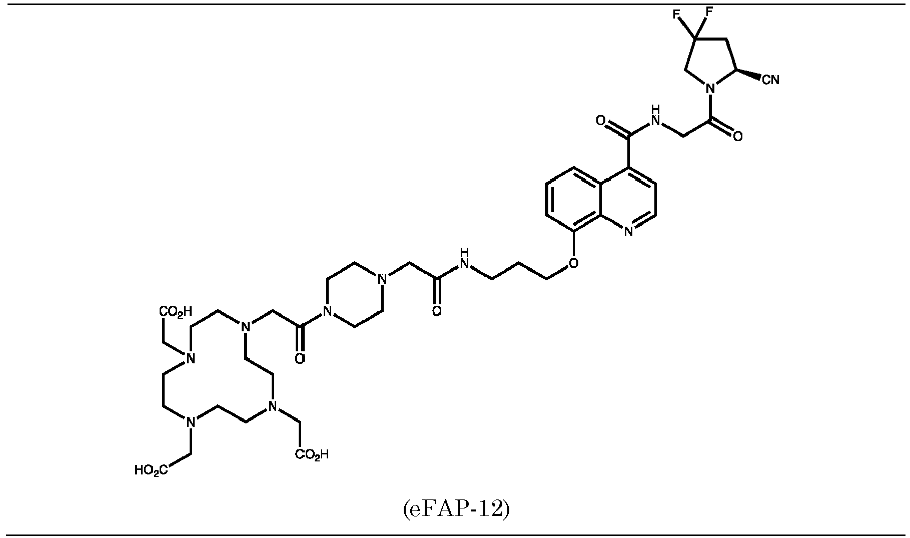

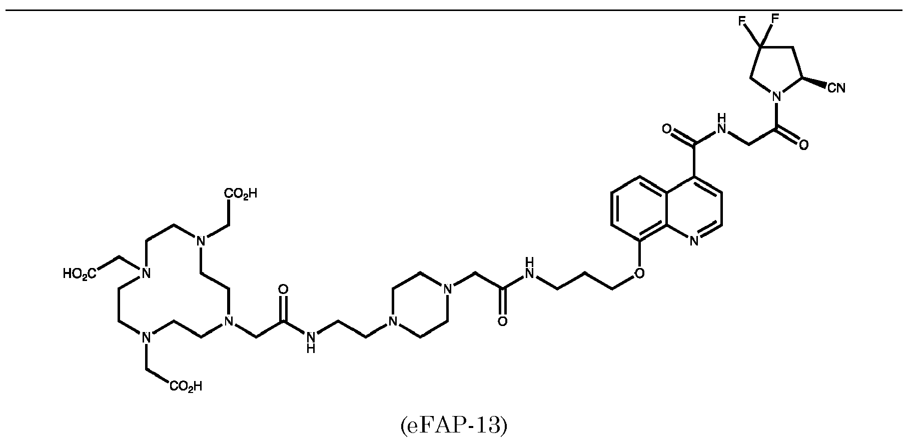

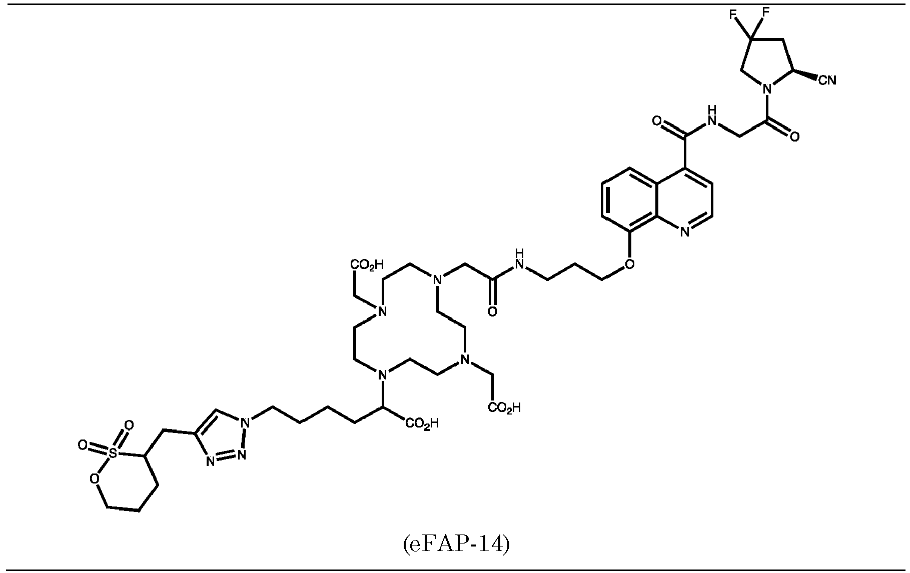

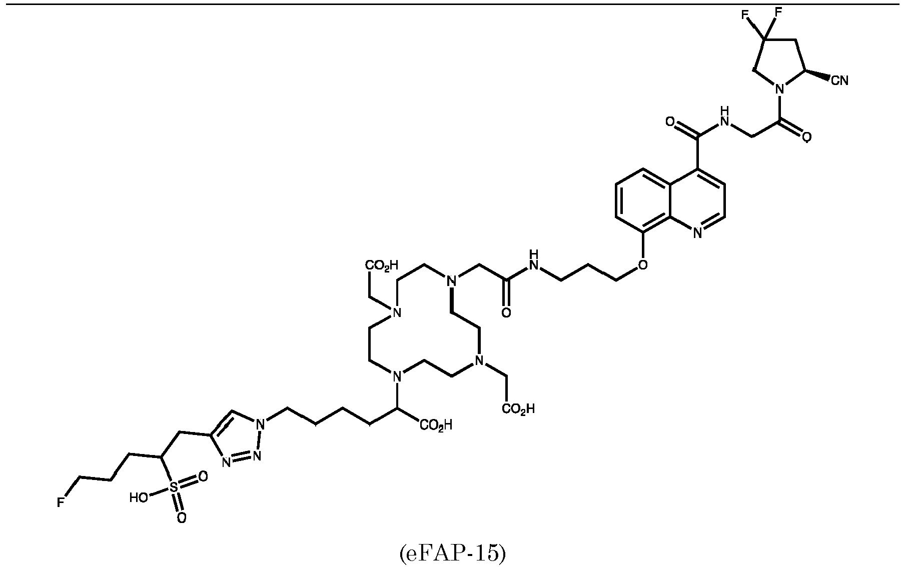

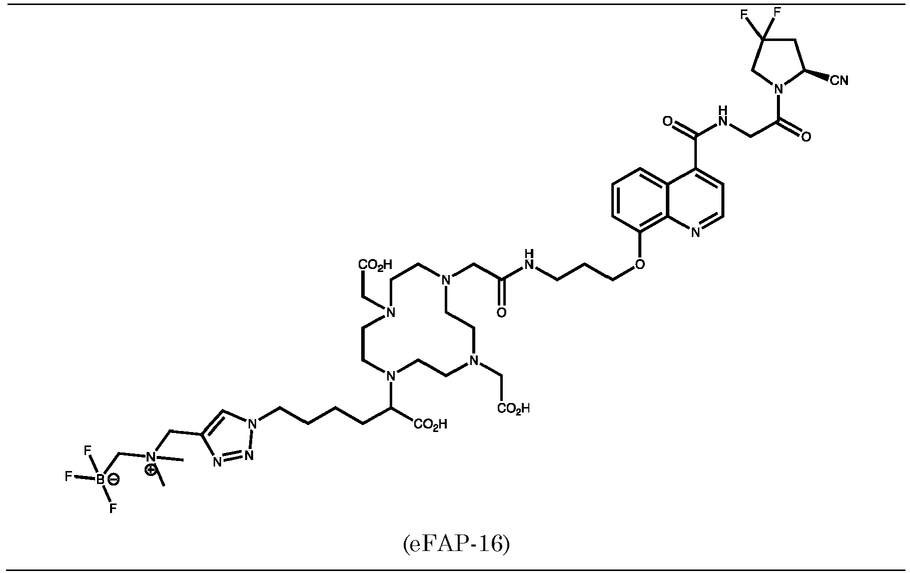

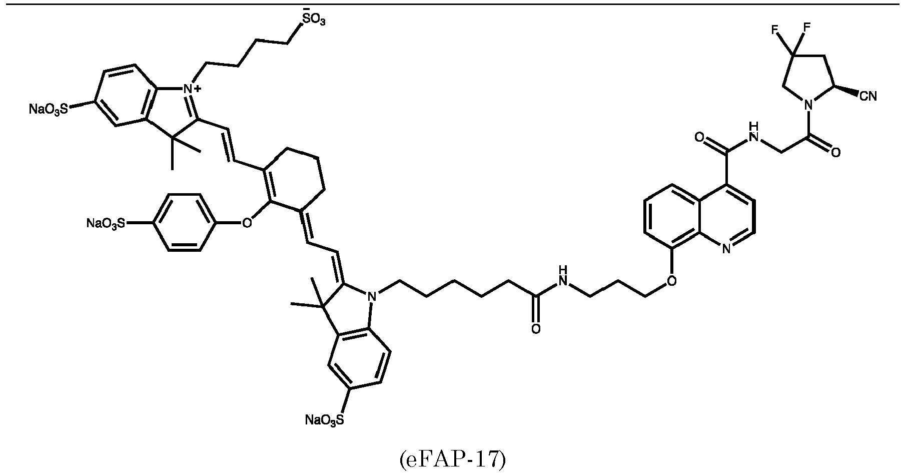

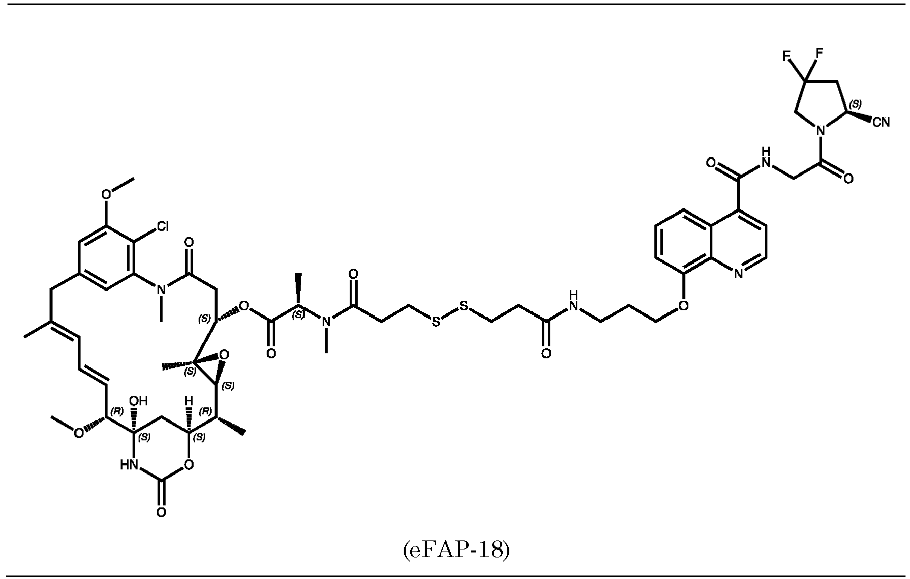

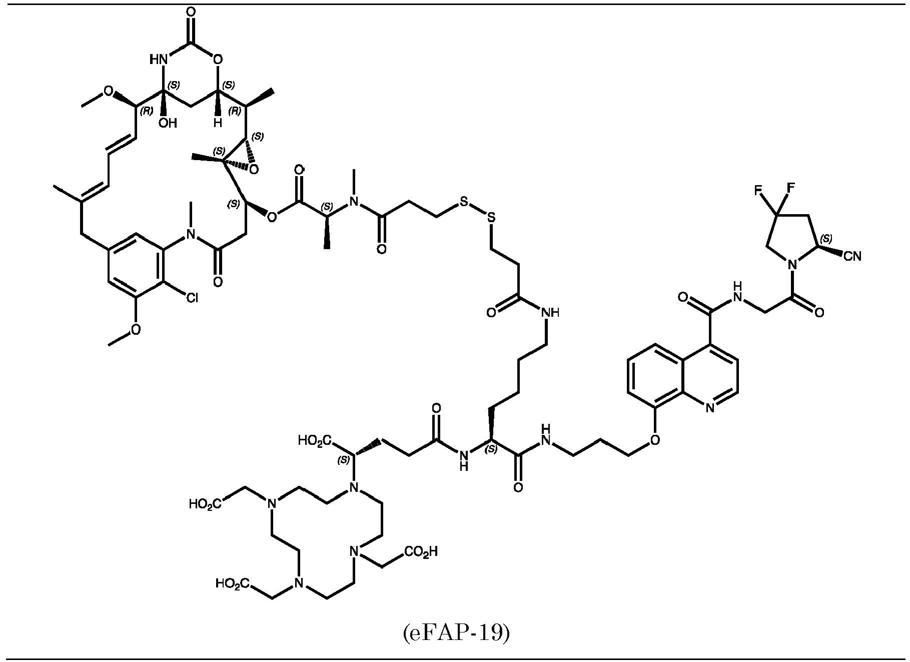

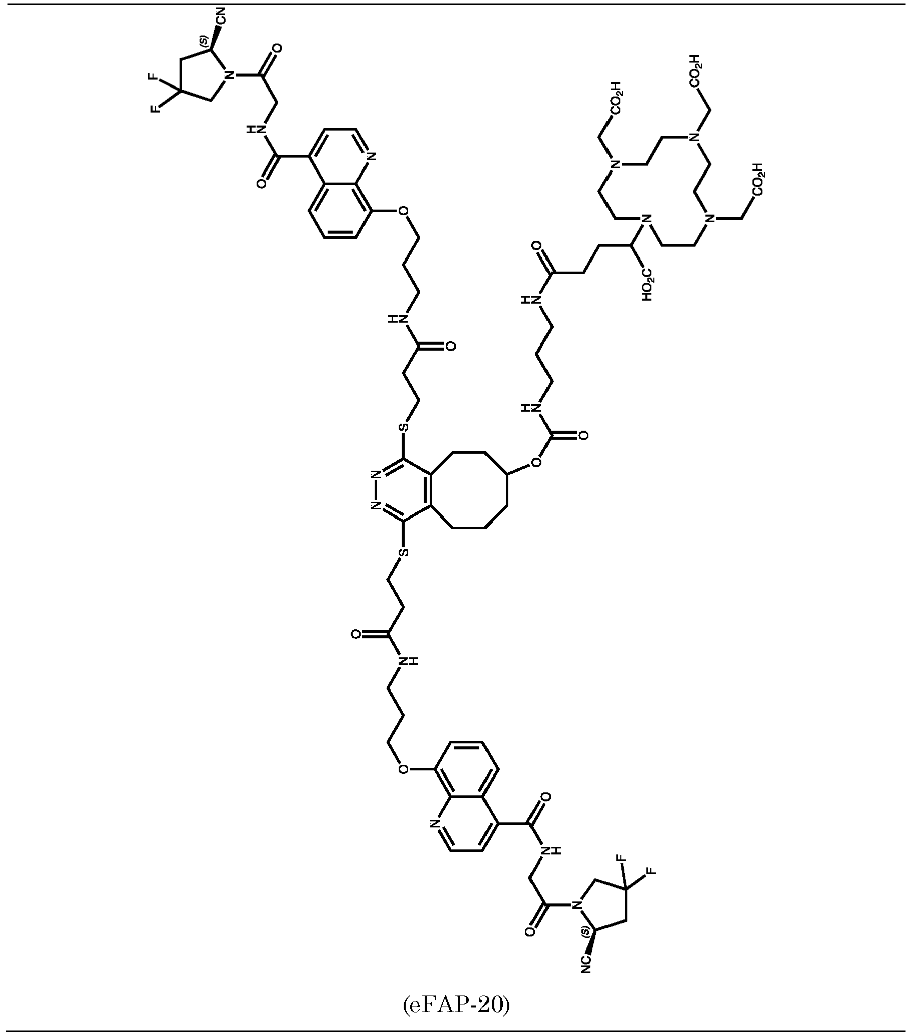

Over 90% of solid tumors contain cancer associated fibroblasts overexpressing FAP. Therefore, the compound of the present invention can be used for the diagnosis and treatment of a broad variety of tumors, including sarcoma, breast, lung, ovarian, head-and-neck, prostate, colorectal, and the like, and even rare or hard-to-treat cancers such as pancreatic and/or brain cancers.

The compound or a pharmaceutically acceptable salt thereof, can be for use as a medicament. More specifically, for use in a medical treatment or diagnosis (including imaging) of tumors. Whether it can be used in treatment or diagnosis mostly depends on the used payload, as described herein.

Accordingly, a further aspect of the present invention is directed to a method for the treatment or diagnosis of tumors, in particular FAP overexpressing tumors. Said method comprises administration of the compound or a pharmaceutically acceptable salt thereof to a patient in a pharmaceutically acceptable dose.

It may be appreciated that for any moiety that may optionally be substituted or interrupted as described herein, such a moiety may also be unsubstituted or uninterrupted.

Furthermore, when a compound is described herein, its salts, prodrugs, metabolites, and the like are also referred to, unless specifically noted otherwise.

When the stereochemistry of an atom in a structure is undefined (*e.g.* C-R¹), the structure refers to all stereoisomers individually of said atom.

As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features.

For the purpose of clarity and a concise description features are described herein as part of the same or separate embodiments, however, it will be appreciated that the scope of the invention may include embodiments having combinations of all or some of the features described.

The present invention can be illustrated by the following non-limited examples and embodiments.

### GENERAL

The chemicals and solvents were purchased from commercial suppliers and used without further purification. Reactions were magnetically stirred and monitored by thin-layer chromatography (TLC) on Merck aluminum-backed precoated plates (silica gel 60 F254). Melting points were determined using a Stuart SMP20. Liquid chromatography-mass spectrometry (LC-MS) was carried out on an Agilent 1260 Infinity II electrospray ionization (ESI) LC-MS system equipped with an Agilent^{®}, InfinityLab Poroshell 120 EC-C18 column (2.7 µm, 3.0 × 100 mm). Products were eluted using a gradient of acetonitrile (ACN; 5 - 100%) in H₂O containing 0.1% formic acid (FA) at a flow rate of 0.5 mL/min for 8 minutes and monitored at 220 nm, 254 nm, and 280 nm by an UV detector. Nuclear magnetic resonance (NMR) spectra were recorded in deuterated dimethyl sulfoxide (DMSO-d₆) and chloroform-d (CDCl₃) on a Bruker AVANCE 400 or a Nanalysis 60 Pro at ambient temperature. Chemical shifts are given as *δ* values in ppm, and coupling constants *J* are given in Hz. The splitting patterns are reported as s (singlet), d (doublet), t (triplet), q (quadruplet), qt (quintuplet), m (multiplet), and br (broad signal). Raw NMR data was processed using MestreNova 13. Purification of the compounds was carried out on a preparative HPLC 1260 Infinity II system from Agilent using a preparative column (50 × 21.2 mm, 5 µm) and a gradient elution of ACN (10% to 95% in H₂O, containing 0.1% FA at a flow rate of 10 mL/min over 10 min.

Instant thin-layer chromatography (iTLC-SG) plates on silica gel impregnated glass fiber sheets were eluted with sodium citrate (0.1 M, pH 5). The plates were analyzed by bSCAN radio-chromatography scanner from Brightspec equipped with a sodium iodide detector. The radioactive samples used for the determination of LogD_{7.4}, *in vitro* assays and *in vivo* studies were counted using a Wizard 2480 gamma counter. Activity measurements were performed using the VDC-405 dose calibrator. Quality control of the radiolabeled compounds, as well as the analysis of their stability, were carried out on an ultra-high performance liquid chromatography (UHPLC) Acquity Arc system from Waters equipped with a diode array detector, a radio-detector from Canberra and an analytical C18 Gemini^{®} column (250.0 × 4.6 mm, 5 µm) eluted with a gradient of ACN (5 to 95% in H₂O, containing 0.1% trifluoroacetic acid TFA) at a flow rate of 1 mL/min over 30 min.

### Preparation of (S)-1-tert-butyl 2-methyl 4,4-difluoropyrrolidine-1,2-dicarboxylate

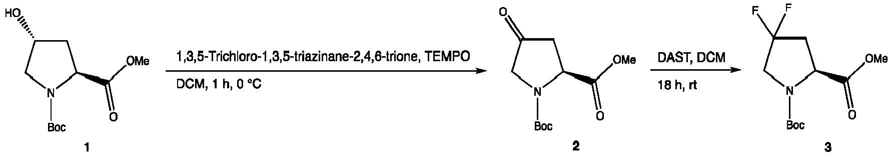

### Step 1: (S)-1-tert-butyl 2-methyl 4-oxopyrrolidine-1,2-dicarboxylate (2)

1,3,5-Trichloro-1,3,5-triazinane-2,4,6-trione (0.95 g, 4.07 mmol) was added to a cooled (0 °C) solution of (S)-1-*tert*-butyl 2-methyl 4-oxopyrrolidine-1,2-dicarboxylate (0.95 g, 3.88 mmol) in DCM (10 ml), followed by the addition of catalytic TEMPO (6 mg, 0.04 mmol). After 5 min the mixture was allowed to reach room temperature, stirred for another 30 minutes and filtrated over Celite. The organic layer was washed with 10 mL saturated potassium carbonate solution, sodium thiosulfate, brine, dried over anhydrous sodium sulfate, filtrated and evaporated. The crude compound 2 (0.65 g, 70%) was used without further purification. ¹H NMR (400 M Hz, CDCl₃): δ 4.77 (dd, 1H, *J* = 36.8, 8 Hz), 3.88 (br s, 2H), 3.75 (s, 3H), 2.90 (s, 1H), 2.57 (dd, 1H, *J* = 18.8, 2.4 Hz), 1.46 (s, 9H). ESI-MS: m/z 376.2 [M + MeOH + H]⁺.

### Step 2: (S)-1-Tert-butyl 2-methyl 4,4-difluoropyrrolidine-1,2-dicarboxylate (3)

A solution of **2** (0.23 g, 0.946 mmol) in DCM (3 mL) was treated with a solution of diethylaminosulfur trifluoride (DAST, 0.197 mL, 1.607 mmol) in DCM (2 mL) at room temperature. Ethanol (0.011 mL, 0.189 mmol) was added and the mixture was stirred for 18 h at rt. The solution was poured into saturated sodium bicarbonate, extracted with DCM (3 × 15 mL), dried (Na₂SO₄), filtered and evaporated in vacuo. Chromatography on silica gel (100% DCM) afforded the product as a yellowish oil (0.150 g, 61%). ¹H NMR (400 MHz, CDCl₃): δ 4.55-4.45 (m, 1H), 3.90-3.60 (m, 2H), 3.75 (s, 3H), 2.81-2.61 (m, 1H), 2.45 (dq, 1H, *J* = 13.6, 5.2 Hz,), 1.44 (br s, 9H). ESI-MS: m/z 266.1 [M + H]⁺.

### Preparation of the intermediate 10

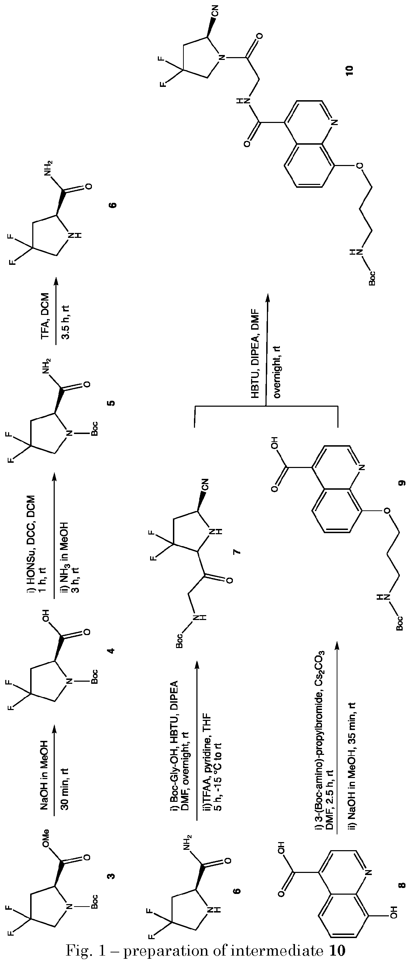

Intermediate 10 was prepared according to the scheme of Figure 1.

### Step 1: (S)-1-(Tert-butoxycarbonyl)-4,4-difluoropyrrolidine-2-carboxylic acid (4)

**3** (2.06 g, 7.78 mmol) was dissolved in MeOH (10 mL) and a solution of NaOH (0.67 g, 17.18 mmol) in MeOH (15 mL) was added dropwise. The reaction mixture was stirred at room temperature for 30 min. Upon completion, the reaction mixture was acidified using IN HCL to pH 2 and the aqueous layer was extracted with DCM (3 × 50 mL). The combined organic layers were washed with brine, dried over MgSO₄ and concentrated in vacuo. It yielded an off-white solid product (1.62 g, 6.44 mmol, 86%). ¹H NMR (60 MHz, DMSO-d₆): *δ* 4.33 (dd, 1H, *J* = 9.3, 4.4 Hz), 3.68 (t, 2H, *J* = 13.2 Hz), 2.76-2.12 (m, 2H), 1.34 (s, 9H). mp: 118-119 _{°}C. ESI-MS: m/z 152.1 [M + H - Boc]⁺.

### Step 2: Tert-butyl (S)-2-carbamoyl-4,4-difluoropyrrolidine-1-carboxylate (5)

**4** (1.55 g, 6.15 mmol) was dissolved in DCM (dry, 30 mL) and HONSu (0.88 g, 7.65 mmol) was added and stirred at room temperature until fully dissolved. DCC (1.55 g, 7.52 mmol) was added and stirred at rt for 30 min as slowly insoluble formed in the reaction mixture. NH₃ (14 mL, 4 M in MeOH) was added dropwise and the reaction mixture was stirred at rt for 3 h. Upon completion, EtOAc (50 mL) was added and the organic layer was washed with sat. aq. NaHCO₃ (5 × 25 mL), brine (25 mL), dried over MgSO₄ and concentrated *in vacuo.* The product was obtained as an off-white solid (1.53 g, 6.02 mmol, 97%). ¹H NMR (60 MHz, CDCl₃): *δ* 6.50 (br, s, 1H), 5.60 (br, s, 1H), 4.48 (dd, 1H, *J* = 8.6, 5.8 Hz), 3.76 (td, 2H, *J* = 12.4, 5.2 Hz), 2.74 (td, 2H, *J* = 13.6, 4.6 Hz), 1.46 (s, 9H). mp: 127-128 _{°}C. ESI-MS: m/z 151.1 [M + H - Boc]⁺.

### Step 3: (S)-4,4-Difluoropyrrolidine-2-carboxamide (6)

**5** (1.19 g, 4.69 mmol) was dissolved in DCM (10 mL) and TFA (5 mL). The reaction mixture was stirred at rt for 2.5 h. Upon completion, all volatiles were removed. Cold ether was added and **6** was recovered as a white solid (0.98 g, 3.66 mmol, 78%). ¹H NMR (60 MHz, DMSO-d₆): *δ* 7.82 (s, 1H), 7.70 (s, 1H), 4.55-4.17 (t, 1H, *J* = 7.8 Hz), 3.54 (d, 2H, *J* = 12.0 Hz), 2.45 (m, 2H). mp: > 130 _{°}C. ESI-MS: m/z 151.0 [M + H]⁺.

### Step 4: Tert-butyl (S)-(2-(2-eyano-4,4-difluoropyrrolidin-1-yl)-2-oxoethyl)carbamate (7)

**6** (0.93 g, 3.47 mmol) was dissolved in DMF (30 mL). Boc-Gly-OH (0.81 g, 4.62 mmol), HBTU (1.64 g, 4.32 mmol) and DIPEA (3.0 mL, 2.3 g, 17.6 mmol) were added and the reaction mixture was stirred overnight. EtOAc (50 mL) was added and the organic layer was washed with brine (5 × 25 mL), dried over MgSO₄ and concentrated in vacuo. The crude reaction mixture was redissolved in THF (30 mL) and cooled to -15 _{°}C. Pyridine (1.00 mL, 12.4 mmol) and TFAA (1.00 mL, 7.19 mmol) were added and the reaction mixture and was stirred for 5 h, allowing to reach rt. EtOAc (50 mL) and H₂O (50 mL) were added. The layers were separated and the aqueous layer was extracted with EtOAc (3 × 25 mL). The combined organic layers were washed with water and brine, dried over MgSO₄ and concentrated *in vacuo.* The product was purified by flash chromatograph (silica gel, Hexane/EtOac 1:1). **7** was obtained as a yellow solid (200 mg, 0.69 mmol, 25%). ¹H NMR (60 MHz, CDCl₃): *δ* 5.48 (s, 1H), 4.94 (t, 1H, *J* = 6.3 Hz), 3.70-4.15 (m, 4H), 2.42-2.94 (m, 2H), 1.40 (s, 9H). mp: 128-129 _{°}C. ESI-MS: m/z 190.1 [M + H - Boc]⁺.

### Step 5: 8-(3-((Tert-butoxycarbonyl)amino)propoxy)quinoline-4-carboxylic acid (9)

8 (0.25 g, 1.32 mmol) was dissolved in DMF (25 mL). 3-(Boc-amino)-propylbromide (0.94 g, 4.0 mmol) and Cs₂CO₃ (1.31 g, 4.0 mmol) were added. The reaction mixture was stirred at rt for 2.5 h. Upon completion, EtOAc (50 mL) was added and the organic layer was washed with brine (5 × 25 mL), dried over MgSO₄ and concentrated *in vacuo.* The residue was redissolved in MeOH (10 mL) and NaOH (78 mg, 2.0 mmol) was added. The reaction mixture was stirred at rt for 35 minutes. The reaction mixture was concentrated *in vacuo* and the product was purified using prep-HPLC. **9** was obtained as a light-yellow solid (0.26 g, 0.75 mmol, 57%). ¹H NMR (60 MHz, DMSO-d₆): *δ* 8.94 (d, 1H, *J* = 4.2 Hz), 8.16 (d, 1H, *J* = 8.3 Hz), 7.88 (d, 1H, *J* = 4.3 Hz), 7.55 (t, 1H, *J* = 8.1 Hz), 7.18 (d, 1H, *J* = 7.4 Hz), 4.16 (t, 2H, *J* = 5.9 Hz), 3.39-2.91 (m, 2H), 1.93 (t, 2H, *J =* 6.1 Hz), 1.32 (s, 9H). mp: > 130 _{°}C. ESI-MS: m/z 346.7 [M + H]⁺.

### Step 6: Tert-butyl (S)-(3-((4-((2-(2-cyano-4,4-difluoropyrrolidin-1-yl)-2-oxoethyl)carbamoyl)quinolin-8-yl)oxy)propyl)carbamate (10)

**7** (0.18 g, 0.61 mmol) was dissolved in DCM (3 mL) and TFA (3 mL). The reaction mixture was stirred at rt for 45 min. Upon completion, all volatiles were removed under a gentle air flow. **9** (0.2 g, 0.58 mmol), HBTU (0.34 g, 0.87 mmol) and DIPEA (0.35 mL) were added to the residue redissolved in DMF (5 mL). The reaction mixture was stirred at rt overnight. EtOAc (50 mL) was added and the organic layer was washed with brine (5 × 25 mL), dried over MgSO₄ and concentrated *in vacuo.* The product was purified using flash chromatography (Silica gel, Hexane/EtOac 1:1) to yield **10** as a light-yellow sticky oil (0.23 g, 0.44 mmol, 72%). ¹H NMR (60 MHz, CDCl₃): *δ* 8.69 (d, 1H, *J* = 4.2 Hz), 7.64 (m, 2H), 7.45-7.02 (m, 2H), 6.86 (d, 1H, *J* = 7.3 Hz), 6.30 (s, 1H), 4.82 (s, 1H), 4.02 (m, 4H), 3.22 (m, 2H), 2.55 (m, 4H), 2.04 (m, 2H), 1.35 (s, 9H). ESI-MS: m/z 618.0 [M + H]⁺.

### Example 1: preparation of (S)-2,2',2"-(10-(2-((3-((4-((2-(2-Cyano-4,4-difluoropyrrolidin-1-yl)-2-oxoethyl)carbamoyl)quinolin-8-yl)oxy)propyl)amino)-2-oxoethyl)-1,4,7,10-tetraazacyclododecane-1,4,7-triyl)triacetic acid (eFAP-6)

**10** (0.02 g, 0.04 mmol) was dissolved in a mixture of DCM (200 µL), TIPS (20 µL) and TFA (200 µL). The reaction mixture was stirred at rt for 30 min. All volatiles were removed under a gentle air flow. The residue was redissolved in DMF (1 mL). DOTA-NHS ester (0.035 g, 0.043 mmol) and DIPEA (45 µL) were added. The reaction mixture was stirred at rt for 30 min and the solvent was removed under reduced pressure. The product was isolated by preparative HPLC to yield eFAP-6 as a light-yellow powder (12.3 mg, 0.015 mmol, 38%). ESI-MS: m/z 804.4 [M + H⁺].

### Example 2 : preparation of 1-(6-((3-((4-((2-((S)-2-Cyano-4,4-difluoropyrrolidin-1-yl)-2-oxoethyl)carbamoyl)quinolin-8-yl)oxy)propyl)amino)-6-oxohexyl)-3,3-dimethyl-5-sulfo-2-((1E,3E)-5-((E)-1,3,3-trimethyl-5-sulfoindolin-2-ylidene)penta-1,3-dien-1-yl)-3H-indol-1-ium (eFAP-7)

**10** (0.01 g, 0.02 mmol) was dissolved in a mixture of DMC (100 µL) and TIPS (10 µL) and TFA (100 µL). The reaction mixture was stirred at rt for 30 min. All volatiles were removed under a gentle air flow. The residue was redissolved in DMF (0.5 mL). Sulfo-Cy5-NHS ester (2.5 mg, 3.2 µmol) and DIPEA (10 µL) were added. The reaction mixture was stirred at rt for 45 min and the solvent was removed under reduced pressure. The product was isolated using preparative HPLC to yield eFAP-7 as a bright blue powder (1.0 mg, 1 µmol). ESI-MS: m/z 1042.6 [M + H]⁺.

### Example 3: preparation of 2,2',2"-(10-(1-Carboxy-4-((3-((4-((2-((S)-2-cyano-4,4-difluoropyrrolidin-1-yl)-2-oxoethyl)carbamoyl)quinolin-8-yl)oxy)propyl)amino)-4-oxobutyl)-1,4,7,10-tetraazacyclododecane-1,4,7-triyl)triacetic acid (eFAP-8)

**10** (10 mg, 9.6 µmol) was dissolved in a solution mixture of DCM/TFA (0.5 mL/0.5 mL). The reaction mixture was stirred at rt for 30 min. All volatiles were removed and the residue was redissolved in DMF (1 mL). DOTA-GA anhydride (5.5 mg, 0.012 mmol) and DIPEA (15 µL) were added, and the reaction mixture was stirred at rt for 2 h. Upon evaporation, eFAP-8 was purified using prep-HPLC to provide a light-yellow powder (8 mg, 9.1 µmol, 95%). ESI-MS: m/z 876.4 [M + H]⁺.

### Example 4: preparation of 2,2',2"-(10-(2-(((4-((3-((4-((2-((S)-2-Cyano-4,4-difluoropyrrolidin-1-yl)-2-oxoethyl)carbamoyl)quinolin-8-yl)oxy)propyl)carbamoyl)cyclohexyl)methyl)amino)-2-oxoethyl)-1,4,7,10-tetraazacyclododecane-1,4,7-triyl)triacetic acid (eFAP-9)

**10** (10 mg, 9.6 µmol) was treated with a solution of DCM/TFA (2 mL, 1:1) at rt for 30 min. All volatiles were removed under a gentle air flow to obtain a brown crude solid. The crude was redissolved in DMF (1 mL), followed by addition of Fmoc-Amcha-OH (2 eq, 7.3 mg), HBTU (4 eq, 14.6 mg), DIPEA (15 µL) and DMF (1 mL). The reaction was stirred at rt for 2 h, diluted with EtOAc (15 mL), washed with water (3 × 5 mL), dried over Na₂SO₄ and concentrated under vacuum to obtain a light-yellow solid. The crude product was used for the next step without further purification. It was redissolved in 20% piperidine in DMF (2 mL) and stirred at rt for 30 min. The solvent was removed under reduced pressure, followed by the addition of DMF (1.5 mL), DOTA-NHS ester (6.6 mg, 14.4 µmol) and DIPEA (15 µL). After 4 h, the solvent was removed under vacuum and the crude product was purified by prep-HPLC to yield eFAP-9 (4.1 mg, 2.4 µmol, 45%) as an off-white powder. ESI-MS: m/z 943.4 [M + H]⁺.

### Example 5: preparation of (S)-2,2',2"-(10-(2-((1-(2-((3-((4-((2-(2-Cyano-4,4-difluoropyrrolidin-1-yl)-2-oxoethyl)carbamoyl)quinolin-8-yl)oxy)propyl)amino)-2-oxoethyl)piperidin-4-yl)amino)-2-oxoethyl)-1,4,7,10-tetraazacyclododecane-1,4,7-triyl)triacetic acid (eFAP-10)

**10** (10 mg, 9.6 µmol) was treated with a solution of DCM/TFA (2 mL, 1:1) at rt for 30 min. All volatiles were removed under a gentle air flow to obtain a brown crude solid. The crude was redissolved in DMF (1 mL), followed by addition of Fmoc-Pip-OH (2 eq, 7.5 mg), HBTU (4 eq, 14.6 mg), DIPEA (15 µL) and DMF (1 mL). The reaction was stirred at rt for 2 h, diluted with EtOAc (15 mL), washed with water (3 × 5 mL), dried over Na₂SO₄ and concentrated under vacuum to obtain a light-yellow solid. The crude product was used for the next step without further purification. It was redissolved in 20% piperidine in DMF (2 mL) and stirred at rt for 30 min. The solvent was removed under reduced pressure, followed by the addition of DMF (1.5 mL), DOTA-NHS ester (6.6 mg, 14.4 µmol) and DIPEA (15 µL). After 4 h, the solvent was removed under vacuum and the crude product was purified by prep-HPLC to yield eFAP-10 (2.1 mg, 2.2 µmol, 23%) as an off-white powder. ESI-MS: m/z 944.4 [M + H]⁺.

### Example 6: preparation of (S)-2,2',2"-(10-(2-((4-(2-(2-((3-((4-((2-(2-Cyano-4,4-difluoropyrrolidin-1-yl)-2-oxoethyl)carbamoyl)quinolin-8-yl)oxy)propyl)amino)-2-oxoethoxy)acetamido)benzyl)amino)-2-oxoethyl)-1,4,7,10-tetraazacyclododecane-1,4,7-triyl)triacetic acid (eFAP-11)

**10** (10 mg, 9.6 µmol) was treated with a solution of DCM/TFA (2 mL, 1:1) at rt for 30 min. All volatiles were removed under a gentle air flow to obtain a brown crude solid. The crude was redissolved in DMF (1 mL), followed by addition of Fmoc-p-Ada-OH (2 eq, 8.7 mg), HBTU (4 eq, 14.6 mg), DIPEA (15 µL) and DMF (1 mL). The reaction was stirred at rt for 4 h, diluted with EtOAc (15 mL), washed with water (3 × 5 mL), dried over Na₂SO₄ and concentrated under vacuum to obtain a light-yellow solid. The crude product was used for the next step without further purification. It was redissolved in 20% piperidine in DMF (2 mL) and stirred at rt for 30 min. The solvent was removed under reduced pressure, followed by the addition of DMF (1.5 mL), DOTA-NHS ester (4.5 mg, 9.6 µmol) and DIPEA (15 µL). After 4 h, the solvent was removed under vacuum and the crude product was purified by prep-HPLC to yield eFAP-11 (2.8 mg, 2.7 µmol, 56%) as an off-white powder. ESI-MS: m/z 1024.4 [M + H]⁺.

### Example 7: preparation of (S)-2,2',2"-(10-(2-(4-(2-((3-((4-((2-(2-Cyano-4,4-difluoropyrrolidin-l-yl)-2-oxoethyl)carbamoyl)quinolin-8-yl)oxy)propyl)amino)-2-oxoethyl)piperazin-1-yl)-2-oxoethyl)-1,4,7,10-tetraazacyclododecane-1,4,7-triyl)triacetic acid (eFAP-12)

**10** (10 mg, 9.6 µmol) was treated with a solution of DCM/TFA (2 mL, 1:1) at rt for 30 min. All volatiles were removed under a gentle air flow to obtain a brown crude solid. The crude was redissolved in DMF (1 mL), followed by addition of Boc-Pipa-OH (2 eq, 4.7 mg), HBTU (4 eq, 14.6 mg), DIPEA (15 µL) and DMF (1 mL). The reaction was stirred at rt for 4 h, diluted with EtOAc (15 mL), washed with water (3 × 5 mL), dried over Na₂SO₄ and concentrated under vacuum to obtain a light-yellow solid. The crude product was used for the next step without further purification. It was redissolved in 20% piperidine in DMF (2 mL) and stirred at rt for 30 min. The solvent was removed under reduced pressure, followed by the addition of DMF (1.5 mL), DOTA-NHS ester (6.6 mg, 14.4 µmol) and DIPEA (15 µL). After 4 h, the solvent was removed under vacuum and the crude product was purified by prep-HPLC to yield eFAP-12 (4.1 mg, 4.4 µmol, 49%) as an off-white powder. ESI-MS: m/z 930.3 [M + H]⁺.

### Example 8: preparation of (S)-2,2',2"-(10-(2-((2-(4-(2-((3-((4-((2-(2-Cyano-4,4-difluoropyrrolidin-1-yl)-2-oxoethyl)carbamoyl)quinolin-8-yl)oxy)propyl)amino)-2-oxoethyl)piperazin-1-yl)ethyl)amino)-2-oxoethyl)-1,4,7,10-tetraazacyclododecane-1,4,7-triyl)triacetic acid (eFAP-13)

**10** (10 mg, 9.6 µmol) was treated with a solution of DCM/TFA (2 mL, 1:1) at rt for 30 min. All volatiles were removed under a gentle air flow to obtain a brown crude solid. The crude was redissolved in DMF (1 mL), followed by addition of Fmoc-ePipa-OH (2 eq, 7.9 mg), HBTU (4 eq, 14.6 mg), DIPEA (15 µL) and DMF (1 mL). The reaction was stirred at rt for 4 h, diluted with EtOAc (15 mL), washed with water (3 × 5 mL), dried over Na₂SO₄ and concentrated under vacuum to obtain a light-yellow solid. The crude product was used for the next step without further purification. It was redissolved in 20% piperidine in DMF (2 mL) and stirred at rt for 30 min. The solvent was removed under reduced pressure, followed by the addition of DMF (1.5 mL), DOTA-NHS ester (6.6 mg, 14.4 µmol) and DIPEA (15 µL). After 4 h, the solvent was removed under vacuum and the crude product was purified by prep-HPLC to yield eFAP-13 (3.8 mg, 4.1 µmol, 43%) as an off-white powder. ESI-MS: m/z 976.3 [M + H]⁺.

### Example 9: preparation of 2,2'-(4-(1-carboxy-5-(4-((2,2-dioxido-1,2-oxathian-3-yl)methy1)-1H-1,2,3-triazol-1-y1)penty1)-10-(2-((3-((4-((2-((S)-2-cyano-4,4-difluoropyrrolidin-1-yl)-2-oxoethyl)carbamoyl)quinolin-8-yl)oxy)propyl)amino)-2-oxoethyl)-1,4,7,10-tetraazacyclododecane-1,7-diyl)diacetic acid (eFAP-14)

**10** (10 mg, 9.6 µmol) was dissolved in a solution mixture of DCM/TFA (0.5 mL/0.5 mL). The reaction mixture was stirred at rt for 30 min. All volatiles were removed and the residue was redissolved in DMF (1 mL). DOTA-K(N₃)-NHS ester (5.5 mg, 12.0 µmol) and DIPEA (15 µL) were added, and the reaction mixture was stirred at rt for 4 h. EtOAc (15 mL) was added, washed with water (3 × 5 mL), dried over Na₂SO₄ and concentrated under vacuum to obtain a light-yellow solid. The crude product was used for the next step without further purification. 3-(Prop-2-yn-1-yl)-1,2-oxathiane 2,2-dioxide (2.1 mg, 12.0 µmol), copper (II) acetate (0.22 mg, 1.2 µmol), sodium ascorbate (0.26 mg, 1.3 µmol) and a solution of acetonitrile and water (1 mL, 1:1) were added and the reaction mixture was stirred at 60 °C for 12 h. The mixture was concentrated under reduced pressure and purified by prep-HPLC to yield eFAP-14 (5.0 mg, 4.7 µmol, 49%) as an off-white powder. ESI-MS: m/z 1076.2 [M + H]⁺.

### Example 10: preparation of 2,2'-(4-(1-carboxy-5-(4-(5-fluoro-2-sulfopentyl)-1H-1,2,3-triazol-1-yl)pentyl)-10-(2-((3-((4-((2-((S)-2-cyano-4,4-difluoropyrrolidin-1-yl)-2-oxoethyl)carbamoyl)quinolin-8-yl)oxy)propyl)amino)-2-oxoethyl)-1,4,7,10-tetraazacyclododecane-1,7-diyl)diacetic acid (eFAP-15)

**10** (10 mg, 9.6 µmol) was dissolved in a solution mixture of DCM/TFA (0.5 mL/0.5 mL). The reaction mixture was stirred at rt for 30 min. All volatiles were removed and the residue was redissolved in DMF (1 mL). DOTA-K(N₃)-NHS ester (5.5 mg, 12.0 µmol) and DIPEA (15 µL) were added, and the reaction mixture was stirred at rt for 4 h. EtOAc (15 mL) was added, washed with water (3 × 5 mL), dried over Na₂SO₄ and concentrated under vacuum to obtain a light-yellow solid. The crude product was used for the next step without further purification. 7-(Fluoro)hept-1-yne-4-sulfonic acid (2.2 mg, 12.0 µmol), copper (II) acetate (0.22 mg, 1.2 µmol), sodium ascorbate (0.26 mg, 1.3 µmol) and a solution of acetonitrile and water (1 mL, 1:1) were added and the reaction mixture was stirred at 60 °C for 12 h. The mixture was concentrated under reduced pressure and purified by prep-HPLC to yield eFAP-14 (3.7 mg, 3.4 µmol, 35%) as an off-white powder. ESI-MS: m/z 1096.2 [M + H]⁺.

### Example 11: preparation of ((((1-(5-(4,10-bis(carboxymethyl)-7-(2-((3-((4-((2-((S)-2-cyano-4,4-difluoropyrrolidin-1-yl)-2-oxoethyl)carbamoyl)quinolin-8-yl)oxy)propyl)amino)-2-oxoethyl)-1,4,7,10-tetraazacyclododecan-1-yl)-5-carboxypentyl)-1H-1,2,3-triazol-4-yl)methyl)dimethylammonio)methyl)trifluoroborate (eFAP-16)

**10** (10 mg, 9.6 µmol) was dissolved in a solution mixture of DCM/TFA (0.5 mL/0.5 mL). The reaction mixture was stirred at rt for 30 min. All volatiles were removed and the residue was redissolved in DMF (1 mL). DOTA-K(N₃)-NHS ester (5.5 mg, 12.0 µmol) and DIPEA (15 µL) were added, and the reaction mixture was stirred at rt for 4 h. EtOAc (15 mL) was added, washed with water (3 × 5 mL), dried over Na₂SO₄ and concentrated under vacuum to obtain a light-yellow solid. The crude product was used for the next step without further purification. N-((Difluoroboranyl)methyl)-*N*,*N*-dimethylprop-2-yn-1-aminium fluoride (2.2 mg, 12.0 µmol), copper (II) acetate (0.22 mg, 1.2 µmol), sodium ascorbate (0.26 mg, 1.3 µmol) and a solution of acetonitrile and water (1 mL, 1:1) were added and the reaction mixture was stirred at 60 °C for 12 h. The mixture was concentrated under reduced pressure and purified by prep-HPLC to yield eFAP-14 (2.3 mg, 2.2 µmol, 23%) as an off-white powder. ESI-MS: m/z 1066.2 [M + H]⁺.

### Example 12 : preparation of 4-(2-((E)-2-((E)-3-(2-((E)-1-(6-((3-((4-((2-((S)-2-Cyano-4,4-difluoropyrrolidin-1-yl)-2-oxoethyl)carbamoyl)quinolin-8-yl)oxy)propyl)amino)-6-oxohexyl)-3,3-dimethyl-5-sulfoindolin-2-ylidene)ethylidene)-2-(4-sulfophenoxy)cyclohex-1-en-1-yl)vinyl)-3,3-dimethyl-5-sulfo-3H-indol-1-ium-1-yl)butane-1-sulfonate (eFAP-17)

**10** (8 mg, 7.7 µmol) was stirred for 30 min at rt in a solution of DCM/TFA (2 mL, 1:1). Then, all volatiles were removed using a gentle air flow to obtain a brown solid. The crude product was dissolved in DMF (1 mL) and treated with DIPEA (10 µL) and pre-activated CW800 dye. CW800 (5 mg, 5 µmol) was dissolved in DMF (1 mL) and activated by treatment with HONSu (5 mg) and DCC (10.3 mg) for 1 h at rt. The reaction mixture was dried and purified by prep-HPLC to obtain eFAP-17 (1.6 mg, 1.1 µmol, 22%) as a green powder. ESI-MS: m/z 1469.4 [M + H]⁺.

### Example 13: preparation of (1⁴S,1⁶S,3²S,3³S,2R,4S,10E,12E,14R)-8⁶-chloro-1⁴-hydroxy-8⁵,14-dimethoxy-3³,2,7,10-tetramethyl-1²,6-dioxo-7-aza-1(6,4)-oxazinana-3(2,3)-oxirana-8(1,3)-benzenacyclotetradecaphane-10,12-dien-4-yl N-(3-((3-((3-((4-((2-((S)-2-cyano-4,4-difluoropyrrolidin-1-yl)-2-oxoethyl)carbamoyl)quinolin-8-yl)oxy)propyl)amino)-3-oxopropyl)disulfaneyl)propanoyl)-N-methyl-L-alaninate(eFAP-18)

**10** (12.1 mg, 23.4 µmol) was deprotected by treatment with TFA/DCM (1:1) for 1 h. The solvents were removed and the residue was dissolved in DMF (4 mL) and then reacted with 3-(tritylthio)propionic acid (32.5 mg, 69.9 µmol), EDC (17.9 mg, 93.2 µmol) and DIPEA (16.3 µL, 93.2 µmol) for 2 h. DMF was removed under high vacuum. The crude product was dissolved in ACN/H₂O (1:1) and purified by prep-HPLC (11.6 mg, 15.5 µmol, 66%). This intermediate was treated with TFA/DCM (1:1) and TES (7.1 mg, 31.0 µmol) for 1 h. The solvents were removed by gentle air stream. To a solution of the product in DMF (1 mL) was added a solution of 2,2'-dithiodipyridine (17.1 mg, 77.5 µmol) in a mixture of DMF (0.5 mL) and acetic acid (0.1 mL). The solution was stirred for 15 minutes. Sodium acetate aq. (1 mL, 0.2 M) was added dropwise to the stirring solution. The reaction was monitored by LCMS and stopped after 1.5 h. Solvents were removed under vacuum, and the crude product dissolved in ACN/H₂O (1:1) before purification by prep-HPLC (5.7 mg, 9.3 µmol, 60%). The product was dissolved in DMF (2.5 mL) and a solution of DM1 (1.7 mg, 16.8 mmol) in PBS (1 mL, pH 7.4) was added. Sodium carbonate aq. (0.5 mL, 3.5 mg/10 mL) was added dropwise to the stirring solution. The reaction was monitored by LCMS. The reaction was stopped after 1 h. The crude product was dissolved in ACN/H₂O (1:1) and purified by prep-HPLC. eFAP-18 was obtained as an off-white solid (3.3 mg, 2.7 µmol, 28%). ESI-MS: m/z 1242.8 [M + H]⁺.

### Example 14: preparation of 2,2',2"-(10-((2S,17S,22S)-22-carboxy-1-(((1⁴S,1⁶S,3²S,3³S,2R,4S,10E,12E,14R)-8⁶-chloro-1⁴-hydroxy-8⁵,14-dimethoxy-3³,2,7,10-tetramethyl-1²,6-dioxo-7-aza-1(6,4)-oxazinana-3(2,3)-oxirana-8(1,3)-benzenacyclotetradecaphane-10,12-dien-4-yl)oxy)-17-((3-((4-((2-((S)-2-cyano-4,4-difluoropyrrolidin-1-yl)-2-oxoethyl)carbamoyl)quinolin-8-yl)oxy)propyl)carbamoyl)-2,3-dimethyl-1,4,11,19-tetraoxo-7,8-dithia-3,12,18-triazadocosan-22-yl)-1,4,7,10-tetraazacyclododecane-1,4,7-triyl)triacetic acid (eFAP-19)

10 (11.9 mg, 23.1 µmol) was deprotected by treatment with TFA/DCM (1:1) for 1 h. The solvents were removed and the residue was dissolved in DMF (4 mL) and then reacted with Fmoc-Lys(Boc)-OH (43.3 mg, 92.4 µmol), EDC (17.7 mg, 92.4 µmol) and DIPEA (16.1 pL, 92.4 µmol) for 5 h. DMF was removed under high vacuum. The crude product was dissolved in ACN/H₂O (1:1) and purified by prep-HPLC (24.2 mg, 16.4 µmol, 71%). This intermediate was treated with TFA/DCM (1:1) for 1 h. The solvents were removed by gentle air stream. The residue was dissolved in DMF (4 mL) and reacted with 3-(tritylthio)propionic acid (17.1 mg, 49.2 µmol), Oxyma Pure (9.3 mg, 65.6 µmol), HATU (24.9 mg, 65.6 µmol) and DIPEA (11.4 pL, 131.2 µmol). The reaction was monitored by LCMS and stopped after 1 h. Fmoc deprotection was carried out by treatment with 20% PIP in DMF for 1 h. DMF was removed under high vacuum and the crude product was purified by prep-HPLC (2.8 mg, 3.2 µmol, 19%). DOTA-GA anhydride (1.9 mg, 4 µmol) and DIPEA (15 µL) were added to a solution of the intermediate in DMF (0.5 mL). The reaction mixture was stirred at rt for 2 h. Upon evaporation, a solution of 2,2'-dithiodipyridine (1.8 mg, 8.0 µmol) in a mixture of DMF (0.5 mL) and acetic acid (0.1 mL) was added. The solution was stirred for 15 minutes. Sodium acetate aq. (1 mL, 0.2 M) was added dropwise to the stirring solution. The reaction was monitored by LCMS and stopped after 2.5 h. Solvents were removed under vacuum, and the crude product dissolved in ACN/H₂O (1:1) before purification by prep-HPLC (1.4 mg, 2.0 µmol, 62%). The product was dissolved in DMF (0.5 mL) and a solution of DM1 (0.4 mg, 4.0 mmol) in PBS (0.5 mL, pH 7.4) was added. Sodium carbonate aq. (0.25 mL, 3.5 mg/10 mL) was added dropwise to the stirring solution. The reaction was monitored by LCMS. The reaction was stopped after 2 h. The crude product was dissolved in ACN/H₂O (1:1) and purified by prep-HPLC. eFAP-19 was obtained as an off-white solid (1.2 mg, 0.7 µmol, 34%). ESI-MS: m/z 1829.2 [M + H]⁺.

### Example 15: preparation of 2,2',2"-(10-(4-((3-((((1,4-bis((3-((3-((4-((2-((S)-2-cyano-4,4-difluoropyrrolidin-1-yl)-2-oxoethyl)carbamoyl)quinolin-8-yl)oxy)propyl)amino)-3-oxopropyl)thio)-5,6,7,8,9,10-hexahydrocycloocta[d]pyridazin-7-yl)oxy)carbonyl)amino)propyl)amino)-1-carboxy-4-oxobutyl)-1,4,7,10-tetraazacyclododecane-1,4,7-triyl)triacetic acid (eFAP-20)

**10** (12.1 mg, 23.4 µmol) was deprotected by treatment with TFA/DCM (1:1) for 1 h. The solvents were removed and the residue was dissolved in DMF (4 mL) and then reacted with 3-(tritylthio)propionic acid (32.5 mg, 69.9 µmol), EDC (17.9 mg, 93.2 µmol) and DIPEA (16.3 pL, 93.2 µmol) for 2 h. DMF was removed under high vacuum. The crude product was dissolved in ACN/H₂O (1:1) and purified by prep-HPLC (11.6 mg, 15.5 µmol, 66%). This intermediate was dissolved in a degassed solution of monosodium phosphate (50 mM, pH 5), followed by the addition of dichlorotetrazine (3 equiv.) in CHCl₃. The two-phases were stirred vigorously for 1 minute. The aqueous phase was collected, and the organic layer was extracted with an additional portion of water. The aqueous fractions were combined and lyophilized. The crude mixture was then purified by prep-HPLC to yield an orange powder after lyophilization (5.1 mg, 4.7 µmol, 60%). eFAP-20 was prepared by reacting the previous dimer with DOTA-GA-TCO (3.6 mg, 1.1 equiv.). Both starting materials were dissolved in H₂O/ACN (1:1) and incubated at 37 °C for 10 min in an Eppendorf tube protected from light. The reaction mixture was purified by preparative HPLC to give eFAP-20 (5.1 mg, 3.0 µmol, 63%). ESI-MS: *m*/*z* 1744.8 [M + H]⁺.

### ANALYSES AND RESULTS

### Radiolabeling

Labelling was performed by adding ¹¹¹InCl₃ or ¹⁷⁷LuCl₃ to the eFAP compounds (1 nmol) dissolved in a mixture of Gz/Asc (50 mM, 10 µL), NaOAc (2.5 M, 1 µL), EtOH (10 µL) and MQ-H₂O (to adjust final volume to a total of 140 µL). The reaction mixture was heated for 20 min at 90 _{°}C. After cooling down, a solution of DTPA (5 µL) was added to complex the remaining free radiometal.

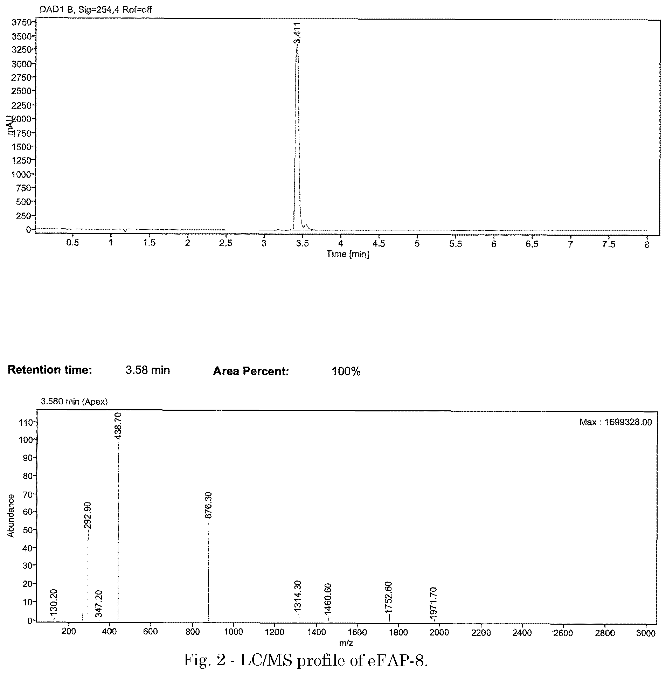

Figure 2 shows the LC/MS profile of eFAP-8.

Figure 3 shows the radio-iTLC chromatogram of [¹¹¹In]In-eFAP-12 showing a radiolabeling yield (RCY) of 97.8%.

### In vitro stability

The radiolabeled compounds (~ 2 MBq) were mixed with 300 µL of PBS (0.1 M, pH 7.4) or mouse serum at 37 °C for 1, 4 and 24 h. At each time point, the proteins in the serum sample were precipitated by addition of acetonitrile (300 µL), and the solution was centrifuged for 5 min at 13,000 rpm (twice). The supernatant was loaded onto a radio-HPLC system for analysis. The samples in PBS were directly analyzed by radio-HPLC without any pre-treatment. All the experiments were performed in triplicate.

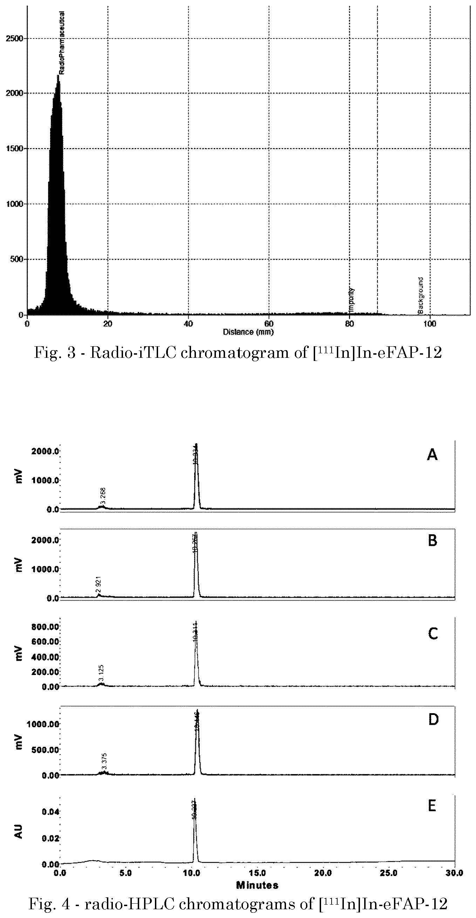

Figure 4 shows radio-HPLC chromatograms of [¹¹¹In]In-eFAP-12 showing the stability of the radiolabeled conjugate over time. A) t = 0 a radiochemical purity (RCP) of 97.4% was obtained, B) RCP = 97% at 2 h, C) RCP = 95.8% at t = 4 h, and D) RCP = 93.6% at t = 24 h. E) The last chromatogram corresponds to the UV signal obtained after injection of ^{nat}In-eFAP-12 confirming the identity of [¹¹¹In]In-eFAP-12.

### LogD_{7.4}

Distribution coefficients (LogD_{7.4} values) were determined by a shake-flask method. Sample containing the ²⁰³Pb-labeled compound was added to a vial containing 600 µL PBS (pH 7.4) and 600 µL n-octanol. The vial was vortexed vigorously and then centrifuged for 10 min for phase separation. Samples of the octanol (200 µL) and aqueous (200 µL) phases were taken and counted on a PerkinElmer WIZARD 2480 gamma counter. LogD_{7.4} value was calculated by using the equation: LogD_{7.4} = log [(counts in octanol phase)/(counts in aqueous phase)]. All the experiments were performed in triplicates.

**Table 1.**

| Distribution coefficients (LogD_{7.4} values) of eFAP-8 to eFAP-13. The eFAP compounds demonstrated a hydrophilic nature. | |
|---|---|
| Compound | LogD_{7.4} |
| eFAP-8 | -3.58 ± 0.09 |
| eFAP-9 | -3.47 ± 0.07 |
| eFAP-10 | -3.64 ± 0.02 |
| eFAP-11 | -3.34 ± 0.07 |
| eFAP-12 | -3.72 ± 0.08 |
| eFAP-13 | -3.28 ± 0.15 |

### In vitro inhibition assay on human and mouse FAP

Enzymatic activity of human FAP (hFAP) and mouse FAP (mFAP) on the Z-GP-AMC substrate was measured at room temperature on a Hidex microplate reader by monitoring the fluorescence at an excitation wavelength of 360 nm and an emission wavelength of 465 nm. The reaction mixture contained 20 µM substrate, 20 nM FAP, assay buffer (50 mM Tris, 100 mM NaCl, 1 mM EDTA, pH 7.4) and tested compound (serial dilution from 10⁻⁶ to 10⁻¹²) in a total volume of 50 µL. The IC₅₀ value is defined as the concentration of inhibitor required to reduce the enzyme activity by 50% after addition of the substrate.

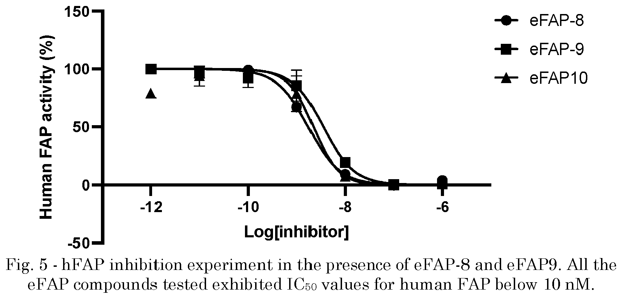

Figure 5 shows the results of hFAP inhibition experiment in the presence of eFAP-8 and eFAP-9. All the eFAP compounds tested exhibited IC₅₀ values for human FAP below 5 nM.

Table 2 shows IC₅₀ values of the other eFAP drugs tested. Results with FAPI-46 obtained in the same assay is included for comparison.

**Table 2.**

| Determination of the IC₅₀ values of the eFAP drugs for human FAP based on an enzymatic assay. | |
|---|---|
| Compound | IC_{50,} human _{FAP} |
| eFAP-6 | 1.6 ± 0.5 |
| eFAP-7 | 2.1 ± 0.6 |
| eFAP-8 | 1.7 ± 0.5 |
| eFAP-9 | 3.6 ± 2.3 |
| eFAP-10 | 2.1 ± 0.3 |
| eFAP-11 | 1.8 ± 0.8 |
| eFAP-12 | 2.1 ± 0.7 |
| eFAP-13 | 1.7 ± 0.5 |
| eFAP-17 | 5.6 ± 1.6 |
| eFAP-18 | 4.4 ± 0.6 |
| FAPI-46 | 2.7 ± 1.7 |

### In vitro inhibition assay on PREP

Enzymatic activity of PREP on the Z-GP-AMC substrate was measured at room temperature on a Hidex microplate reader by monitoring the fluorescence at an excitation wavelength of 360 nm and an emission wavelength of 465 nm. The reaction mixture contained 20 µM substrate, 2 nM PREP, assay buffer (50 mM Tris, 100 mM NaCl, 1 mM EDTA, pH 7.4) and tested compound (serial dilution from 10-6 to 10-12) in a total volume of 50 µL. The IC₅₀ value is defined as the concentration of inhibitor required to reduce the enzyme activity by 50% after addition of the substrate.

The results are given in Table 3. Results with FAPI-46 obtained in the same assay is included for comparison.

### In vitro inhibition assay on DPP4

Enzymatic activity of PREP on the Z-GP-pNA substrate was measured at room temperature on a Hidex microplate reader by monitoring the fluorescence at an excitation wavelength of 380 nm and an emission wavelength of 425 nm. The reaction mixture contained 20 µM substrate, 0.5 nM PREP, assay buffer (50 mM Tris, 100 mM NaCl, 1 mM EDTA, pH 7.4) and tested compound (serial dilution from 10-6 to 10-12) in a total volume of 100 µL. The IC₅₀ value is defined as the concentration of inhibitor required to reduce the enzyme activity by 50% after addition of the substrate.

The results are given in Table 3. Results with FAPI-46 obtained in the same assay is included for comparison.

**Table 3.**

| Determination of the IC₅₀ values of eFAP-6 for murine FAP, PREP and DPP4 based on an enzymatic assay. Binding of eFAP-6 is specific to FAP since the IC₅₀ value of eFAP-6 for PREP was 68-fold higher than the value found for human FAP | | | |
|---|---|---|---|
| Compound | FAPI-46 | eFAP-6 | eFAP-7 |
| IC_{50,} murine | | | |
| _{FAP} | 15.0 ± 2.0 | 3.1 ± 2.4 | 1.7 ± 0.5 |
| IC_{50, PREP} | | 108 ± 42 | |
| IC_{50, DPP4} | | >10'000 | |

### Competitive binding assay in cell cultures

The U-87 MG glioblastoma was cultured at 37 °C and 5% CO₂ in Dulbecco's Modified Eagle Medium (DMEM) supplemented with 10 % (v/v) fetal calf serum. HT-1080.hFAP cells were kept in culture in DMEM medium supplemented with fetal bovine serum (10%, FBS) and antibiotic antimycotic (1%, AA) at 37 °C and 5% CO₂. For passaging, cells were detached using Trypsin-EDTA 0.05% when reaching 90% confluence and re-seeded at a dilution of 1:4.

Competition binding experiments were conducted by incubation of the [¹¹¹In]In-FAPI-46 radioligand with increasing concentrations of eFAP compound in HT-1080.hFAP cells at 37 °C for 1 h. Cells were then washed three times with binding buffer and lysed. The cell-associated radioactivity was measured in a γ-counter. The IC₅₀ values were calculated by fitting the data by non-linear regression using GraphPad Prism.

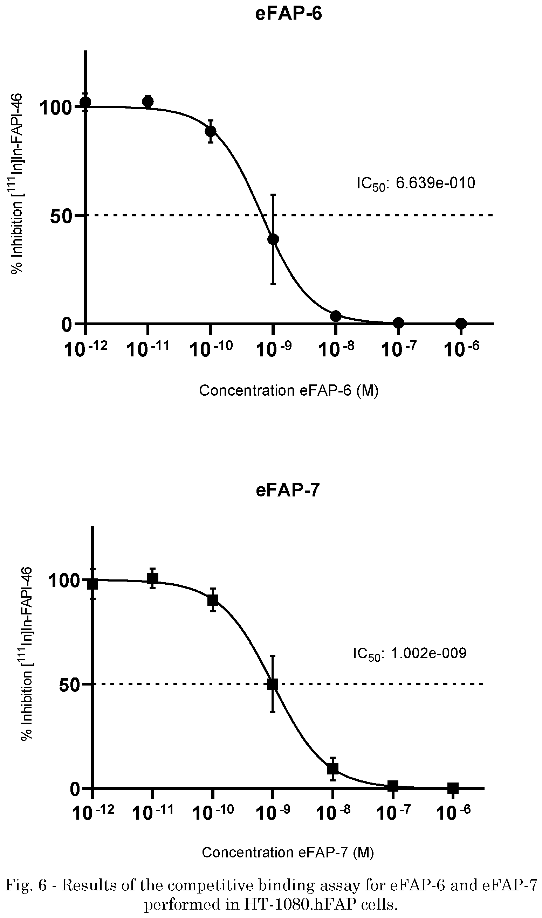

Results of the competitive binding assay for eFAP-6 and eFAP-7 performed in HT-1080.hFAP cells are given in Figure 6 and Table 4. Both compounds showed low nano-molar binding affinity for FAP. Results with FABI-46 obtained in the same assay is included for comparison.

**Table 4.**

| Determination of the binding affinity of eFAP-6 and eFAP-7 for FAP in a cell-based assay. Both compounds displayed a lower IC₅₀ value for FAP than the reference FAP inhibitor FAPI-46. | | | |
|---|---|---|---|
| Compound | eFAP-6 | eFAP-7 | FAPI-46 |
| IC₅₀ | 0.66 | 1.00 | 1.6 ± 0.3 |

### Cytotoxicity assay

In 96-well plates, U-87 cells (3 × 10³) were seeded in each well and incubated at 37 °C and 5% CO₂ for 24 h. The medium was removed and the wells were washed once with PBS. The cytotoxic drug (positive control) or the eFAP conjugate containing the drug were prepared at various concentrations (i.e., 1, 10, 100, 1000 nM) in DMEM culture medium, supplemented with 2 mM L-glutamine, 10% FBS, 50 units/mL penicillin, and 50 µg/mL streptomycin. Medium was used as a negative control. Cells were incubated with 100 µL of the tested compounds for 1, 24, 48, and 72 h. After incubation, medium was removed and each well was washed once with PBS. Next, 100 µL of 10% resazurin solution in culture medium was added to each well and incubated for 2.5 h. Fluorescence measurements were carried out using a microplate reader at 544 nm excitation and 590 nm emission.

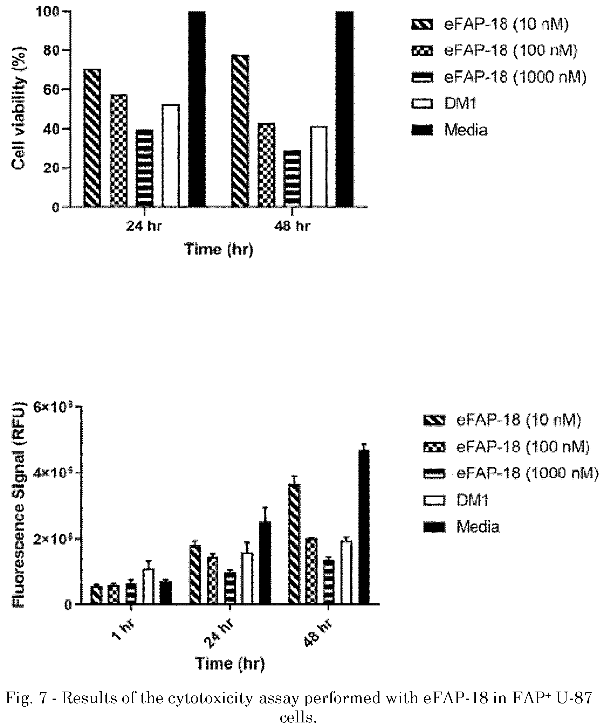

Results of the cytotoxicity assay performed with eFAP-18 in FAP⁺ U-87 cells are given in Figure 7. When the cells were treated with a single dose of eFAP-18 (1 µM) the cell viability decreased to approximately 25% compared to 40% when the cells were incubated with the parent drug DM1.

### Biodistribution studies in animal models

Six-week-old male Balb/c nu/nu-specific and opportunistic pathogen-tree (SOPF) mice were housed in individually ventilated cages, with 4 mice per cage. Upon arrival, mice were acclimated for 1 week, with access to food and water ad libitum. Mice were subcutaneously inoculated on the right shoulder with U-87 or HT-1080.hFAP cells (1 × 10⁶ cells). Xenografts were allowed to grow until they reached an appropriate size at the start of the studies. All animal experiments were approved by the Animal Welfare Committee of the Erasmus MC and were conducted in agreement with institutional guidelines (license number: AVD101002017867).

Biodistribution studies were performed to determine tumor and organ uptake of the radiolabeled eFAP compounds. Animals (n = 4 for each time point and each compound) were injected intravenously with an average of 100 kBq/0.5 nmol at t=0. At 3 selected time points (1, 4 and 24 h) p.i. blood was collected via cardiac puncture under isoflurane/O₂ anesthesia, after which the mice were sacrificed. The tumor and organs of interest (prostate, pancreas, spleen, gall bladder, liver, stomach, small intestine, coecum, colon, adrenals, kidney, lung, heart, salivary glands, muscle, bone, brain were excised, washed in PBS and blotted dry. The blood, tumor, and relevant organs were weighed and measured in a γ-counter. To determine the total injected radioactivity per animal, calibration curve with indium-111 and lutetium-177 were determined. The percentage of injected dose per gram (% ID/g) was determined for each tissue sample and corrected for both the injected volume and % ID present at the injection site (the tail).

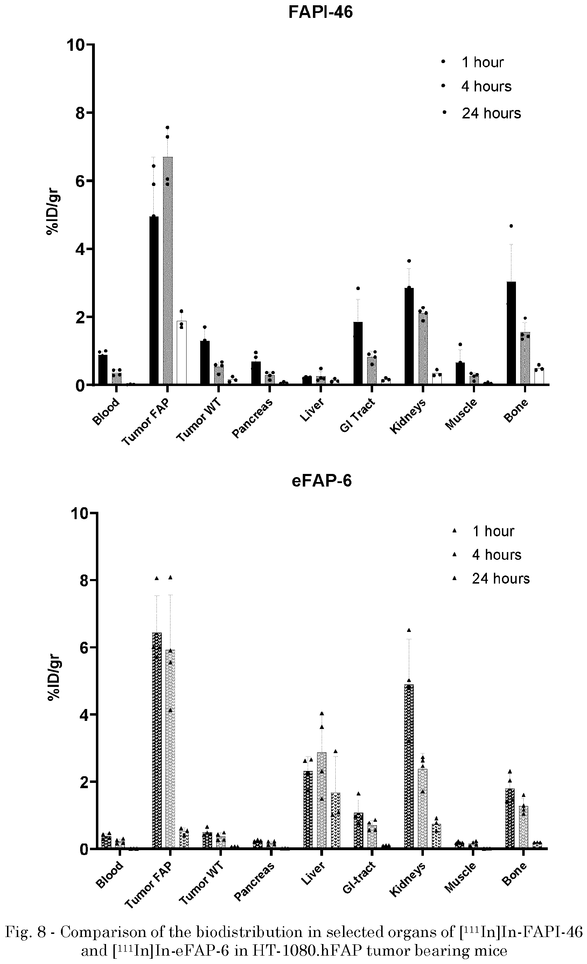

Comparison of the biodistribution in selected organs of [¹¹¹In]In-FAPI-46 and [¹¹¹In]In-eFAP-6 in HT-1080.hFAP tumor bearing mice are given in Figure 8. In vivo biodistribution studies with indium-111 labelled eFAP-6 showed a high and rapid tumor uptake and a low background uptake in healthy organs, resulting in high-contrast images. The tumor targeting performance was comparable with that of FAPI-46, the clinical reference for FAP-targeted tracers. A rapid clearance from the body was observed with eFAP-6, which is ideal for imaging purposes.

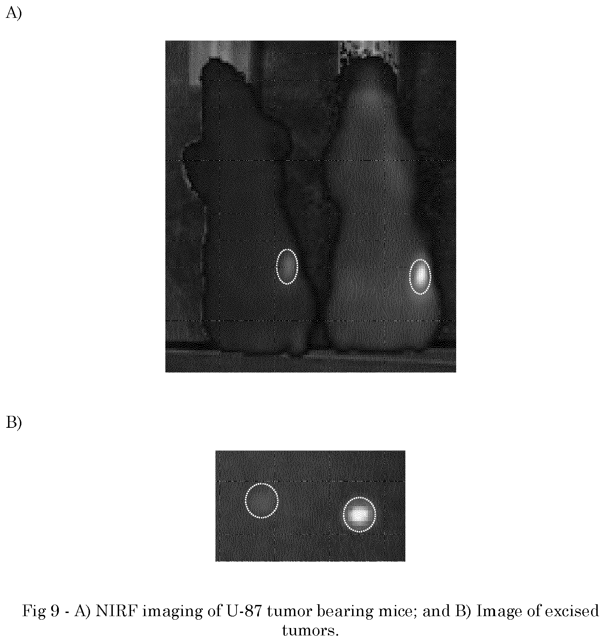

Evaluation of targeting performance of eFAP-17 in near-infrared fluorescence imaging of U-87 tumor bearing mice after intravenous injection of 2 nmol of the probe are given in Figure 9 showing A) NIRF imaging of U-87 tumor bearing mice at 1 h after intravenous injection of 2 nmol of CW800 (left, negative control) or eFAP-17 (right) and B) image of excised tumors 2.5 h post administration of CW800 (left) or eFAP-17 (right). Tumors are indicated by the white circles. eFAP-17 showed a high uptake in the FAP overexpressing tumor and low accumulation in healthy tissues.

## Claims

1. Compound, or a pharmaceutically acceptable salt thereof, comprising a scaffold according to formula (I) 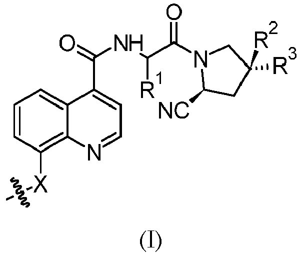 wherein
X represents CH₂ or O;
R¹ represents H, Me, CH(CH₃)C₂H₅, CH₂CH(CH₃)₂, CH(CH₃)₂, CH₂OH, CH₂SH, CH(OH)CH₃, CH₂C(O)NH₂, CH₂CH₂C(O)NH₂, (CH₂)ₘCO₂H, (CH₂)ₘNH₂, wherein m is 1-4; and
R² and R³ each independently represent H or F.

2. Compound according to claim 1, or a pharmaceutically acceptable salt thereof, having a structure according to formula (II) 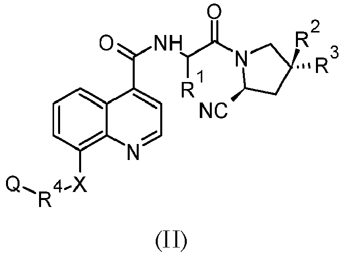 wherein
R⁴ represents a spacer, preferably an aliphatic spacer; and
Q comprises a payload such as a therapeutic agent, a diagnostic agent or a combination thereof, preferably a radioisotope, a fluorescent dye, a drug or a combination thereof.

3. Compound according to claim 2, or a pharmaceutically acceptable salt thereof, having a structure according to formula (IIIa), preferably according to (IIIb) 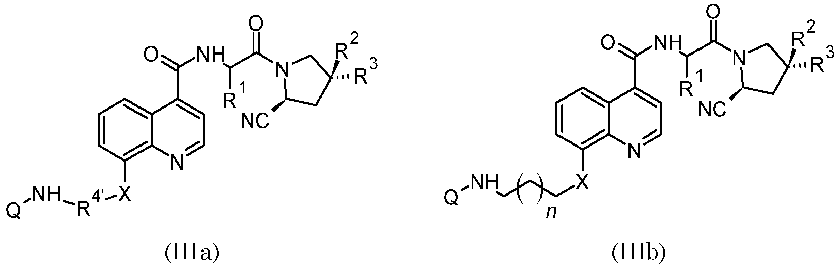 wherein R^{4'} represents an optionally substituted hydrocarbylene or an optionally substituted alkylene ether, preferably an alkylene, more preferably C₁-C₈ alkylene; and n is 1-6, preferably 1.

4. Compound according to any of claims 2-3, or a pharmaceutically acceptable salt thereof, wherein Q comprises one or more of radioisotopes complexed to chelators, fluorescent dyes, and drugs such as a cytotoxic or cytostatic agents.

5. Compound according to any of the previous claims, or a pharmaceutically acceptable salt thereof, wherein said compound has a structure according to formula (IV) 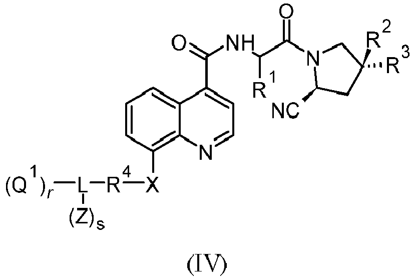 wherein L represent a linker, Q¹ comprises at least one payload, Z comprises an additional scaffold according to formula (I), *r* is 1 or more and s is 0 or more.

6. Compound according to the previous claim, wherein the linker comprises cleavable, non-cleavable sections, or combinations thereof, preferably wherein the linker is based on or comprises linear or branched amino acids such as glycine, alanine, β-alanine,3-aminopropionic acid, 4-aminobutyric acid, 5-aminovaleric acid, 6-aminohexanoic acid, 7-aminoheptanoic acid, 8-aminooctanoic acid, 9-aminononanoic acid, 10-aminodecanoic acid, 2-aminooctanoic acid, and the like, a peptide spacer (Xaa)₁₋₄, wherein each Xaa is independently a proteinogenic or non-proteinogenic amino acid residue selected from the group consisting of a D-amino acid of a proteinogenic amino acid, *N^{ε},N^{ε},N^{ε}*-trimethyl-lysine, 2,3-diaminopropionic acid (Dap), 2,4-diaminobutyric acid (Dab), ornithine (Orn), homoarginine (hArg), 2-amino-4-guanidinobutyric acid (Agb), 2-amino-3-guanidinopropionic acid (Agp), β-alanine,4-aminobutyric acid, 5-aminovaleric acid, 6-aminohexanoic acid, 7-aminoheptanoic acid, 8-aminooctanoic acid, 9-aminononanoic acid, 10-aminodecanoic acid, 2-aminooctanoic acid, 2-aminoadipic acid (2-Aad), 3-aminoadipic acid (3-Aad), 4-(aminomethyl)cyclohexane-1-carbonyl (Amcha), 4-amino-1-carboxymethyl-piperidinyl (Pip), cysteic acid, diglycolic acid, and NH₂(CH₂)₂[O(CH₂)₂]_{q}C(O)OH (wherein q = 1-36), more preferably wherein the linker comprises a moiety having a structure as shown in any of formulae La-Ln; 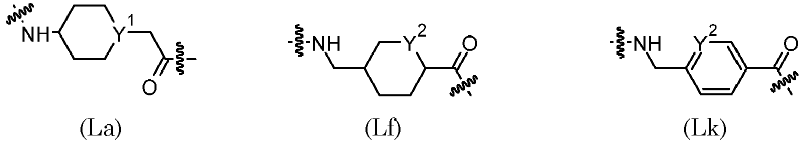 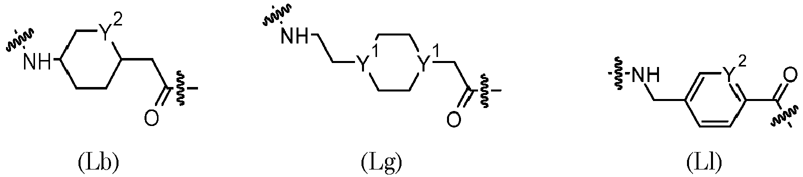 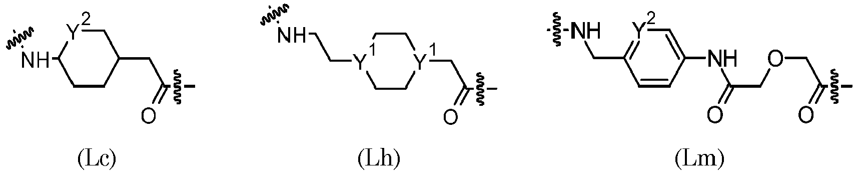 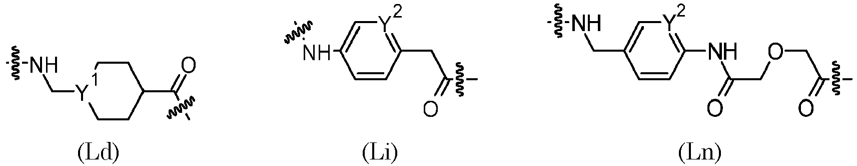 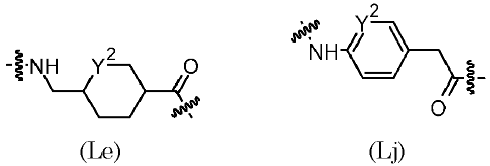 wherein
Y¹ is selected from the group consisting of C and N, preferably N for formula La, Lg and Lh and C for formula Ld; and
Y² is selected from the group consisting of C, N, and O, preferably N and C; more preferably C, even more preferably wherein the linker comprises a moiety having a structure according to any of formulae Laa, Lfa, Lga, Lha, and Lma

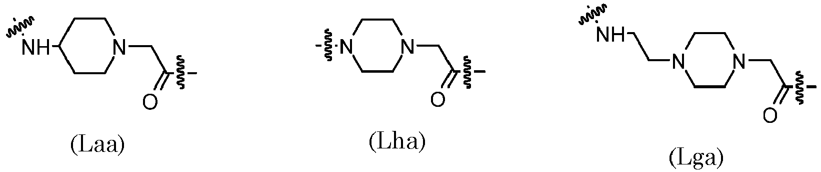

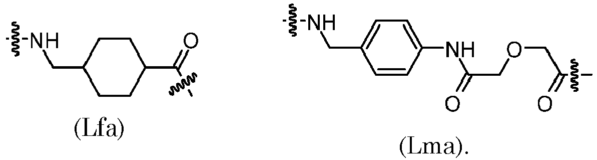

7. Compound according to any of claims 5-6, or a pharmaceutically acceptable salt thereof, wherein the linker L comprises a cleavable linker section comprising an *in vivo* cleavable moiety and optionally an self-immolative moiety, preferably an *in vivo* cleavable moiety selected from the group consisting of amides, ester, carbamates, hydrazones, thiazolidine, methylene alkoxy carbamate, disulfides and combinations thereof, more preferably wherein the *in vivo* cleavable moiety comprises a disulfide moiety or a terminal thiol that enables disulfide linkage to the payload that comprises a terminal thiol as well, most preferably wherein the linker comprises a moiety having the structure according to formula Lo 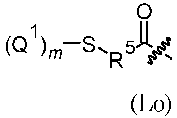 wherein R⁵ represents an optionally present second spacer, preferably an optionally substituted second aliphatic spacer, more preferably a C₁-C₆ alkylene, most preferably an ethylene.

8. Compound according to any of claims 2-7, or a pharmaceutically acceptable salt thereof, wherein Q comprises at least two of a radioisotope, a fluorescent dye and drug, preferably at least a radioisotope and a fluorescent dye or at least a radioisotope and a drug.

9. Compound according to any of claims 2-8, or a pharmaceutically acceptable salt thereof, having a structure according to formula (V) 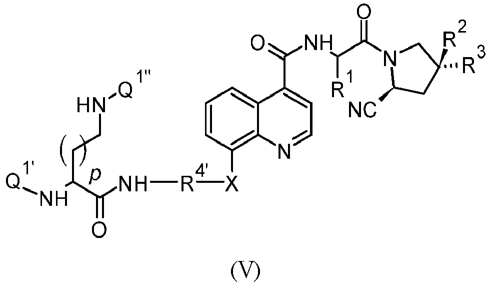 wherein p is 1-6, preferably 3;
Q^{1'} and Q^{1"} each independently comprise a payload such as a radioisotope, a fluorescent dye, or a drug.

10. Compound according to any of claims 5-8, or a pharmaceutically acceptable salt thereof, wherein the linker is linking the scaffold according to formula (I) with one additional scaffold, wherein the linker comprises a multimeric moiety according to any of formulae (Lp) to (Lv) 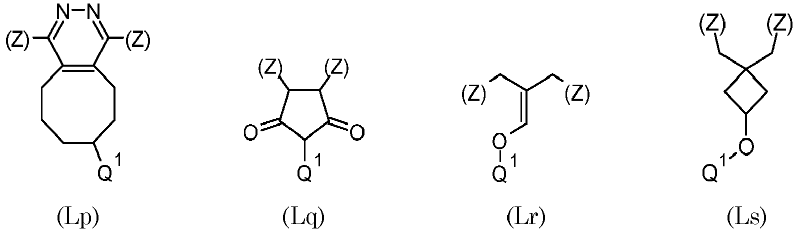 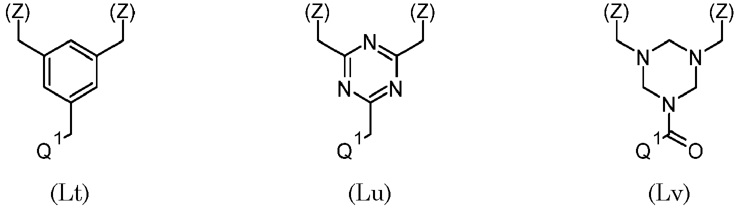 which formulae each show the position of the scaffolds (Z) and the payload Q¹ relative to each other with respect to the multimeric moiety.

11. Compound according to claim 1, or a pharmaceutically acceptable salt thereof, suitable for the preparation of any of the compounds according to any of claims 2-10, said compound having a structure according to formula (VIIa), preferable according to formula (VIIb) 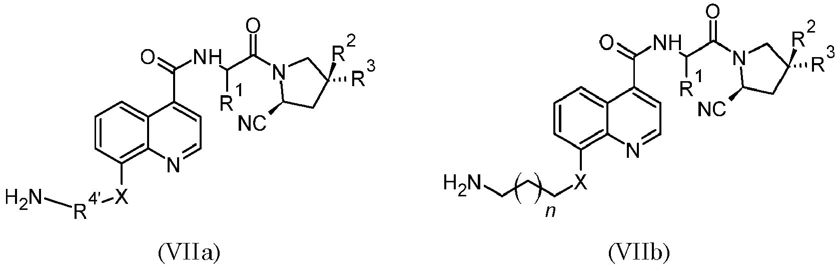 wherein R^{4'} represents an optionally substituted hydrocarbylene or an optionally substituted alkylene ether; and n is 1-6, preferably 1.

12. Compound, or a pharmaceutically acceptable salt thereof, according to any of the previous claims wherein X represents O.

13. Compound, or a pharmaceutically acceptable salt thereof, according to any of the previous claims wherein X represents O, R¹ represents H; and/or at least one of R² and R³ represents F, preferably both R² and R³ represent F.

14. Compound, or a pharmaceutically acceptable salt thereof, according to any of the previous claims, selected from the group consisting of
- (S)-2,2',2"-(10-(2-((3-((4-((2-(2-cyano-4,4-difluoropyrrolidin-1-yl)-2-oxoethyl)carbamoyl)quinolin-8-yl)oxy)propyl)amino)-2-oxoethyl)-1,4,7,10-tetraazacyclododecane-1,4,7-triyl)triacetic acid (eFAP-6);
- 1-(6-((3-((4-((2-((*S*)-2-Cyano-4,4-difluoropyrrolidin-1-yl)-2-oxoethyl)carbamoyl)quinolin-8-yl)oxy)propyl)amino)-6-oxohexyl)-3,3-dimethyl-5-sulfo-2-((1E,3E)-5-((E)-1,3,3-trimethyl-5-sulfoindolin-2-ylidene)penta-1,3-dien-1-yl)-3H-indol-1-ium (eFAP-7)
- 2,2',2"-(10-(1-Carboxy-4-((3-((4-((2-((*S*)-2-cyano-4,4-difluoropyrrolidin-1-yl)-2-oxoethyl)carbamoyl)quinolin-8-yl)oxy)propyl)amino)-4-oxobutyl)-1,4,7,10-tetraazacyclododecane-1,4,7-triyl)triacetic acid (eFAP-8)
- 2,2',2"-(10-(2-(((4-((3-((4-((2-((*S*)-2-Cyano-4,4-difluoropyrrolidin-1-yl)-2-oxoethyl)carbamoyl)quinolin-8-yl)oxy)propyl)carbamoyl)cyclohexyl)methyl)amino)-2-oxoethyl)-1,4,7,10-tetraazacyclododecane-1,4,7-triyl)triacetic acid (eFAP-9)
- (*S*)-2,2',2"-(10-(2-((1-(2-((3-((4-((2-(2-Cyano-4,4-difluoropyrrolidin-1-yl)-2-oxoethyl)carbamoyl)quinolin-8-yl)oxy)propyl)amino)-2-oxoethyl)piperidin-4-yl)amino)-2-oxoethyl)-1,4,7,10-tetraazacyclododecane-1,4,7-triyl)triacetic acid (eFAP-10)
- (*S*)-2,2',2"-(10-(2-((4-(2-(2-((3-((4-((2-(2-Cyano-4,4-difluoropyrrolidin-1-yl)-2-oxoethyl)carbamoyl)quinolin-8-yl)oxy)propyl)amino)-2-oxoethoxy)acetamido)benzyl)amino)-2-oxoethyl)-1,4,7,10-tetraazacyclododecane-1,4,7-triyl)triacetic acid (eFAP-11)
- (*S*)-2,2',2"-(10-(2-(4-(2-((3-((4-((2-(2-Cyano-4,4-difluoropyrrolidin-1-yl)-2-oxoethyl)carbamoyl)quinolin-8-yl)oxy)propyl)amino)-2-oxoethyl)piperazin-1-yl)-2-oxoethyl)-1,4,7,10-tetraazacyclododecane-1,4,7-triyl)triacetic acid (eFAP-12)
- (*S*)-2,2',2"-(10-(2-((2-(4-(2-((3-((4-((2-(2-Cyano-4,4-difluoropyrrolidin-1-yl)-2-oxoethyl)carbamoyl)quinolin-8-yl)oxy)propyl)amino)-2-oxoethyl)piperazin-1-yl)ethyl)amino)-2-oxoethyl)-1,4,7,10-tetraazacyclododecane-1,4,7-triyl)triacetic acid (eFAP-13)
- 2,2'-(4-(1-carboxy-5-(4-((2,2-dioxido-1,2-oxathian-3-yl)methyl)-1*H*-1,2,3-triazol-1-yl)pentyl)-10-(2-((3-((4-((2-((*S*)-2-cyano-4,4-difluoropyrrolidin-1-yl)-2-oxoethyl)carbamoyl)quinolin-8-yl)oxy)propyl)amino)-2-oxoethyl)-1,4,7,10-tetraazacyclododecane-1,7-diyl)diacetic acid (eFAP-14);
- 2,2'-(4-(1-carboxy-5-(4-(5-fluoro-2-sulfopentyl)-1*H*-1,2,3-triazol-1-yl)pentyl)-10-(2-((3-((4*-*((2-((*S*)-2-cyano-4,4-difluoropyrrolidin-1-yl)-2-oxoethyl)carbamoyl)quinolin-8-yl)oxy)propyl)amino)-2-oxoethyl)-1,4,7,10-tetraazacyclododecane-1,7-diyl)diacetic acid (eFAP-15),
- ((((1-(5-(4,10-bis(carboxymethyl)-7-(2-((3-((4-((2-((*S*)-2-cyano-4,4-difluoropyrrolidin-1-yl)-2-oxoethyl)carbamoyl)quinolin-8-yl)oxy)propyl)amino)-2-oxoethyl)-1,4,7,10-tetraazacyclododecan-1-yl)-5-carboxypentyl)-1*H*-1,2,3-triazol-4-yl)methyl)dimethylammonio)methyl)trifluoroborate (eFAP-16)
- 4-(2-((*E*)-2-((*E*)-3-(2-((*E*)-1-(6-((3-((4-((2-((*S*)-2-Cyano-4,4-difluoropyrrolidin-1-yl)-2-oxoethyl)carbamoyl)quinolin-8-yl)oxy)propyl)amino)-6-oxohexyl)-3,3-dimethyl-5-sulfoindolin-2-ylidene)ethylidene)-2-(4-sulfophenoxy)cyclohex-1-en-1-yl)vinyl)-3,3-dimethyl-5-sulfo-3*H*-indol-1-ium-1-yl)butane-1-sulfonate (eFAP-17)
- (1⁴*S*,1⁶*S*,3²*S*,3³*S*,2*R*,4*S*,10*E*,12E, 14*R*)-8⁶-chloro-1⁴-hydroxy-8⁵, 14-dimethoxy-3³,2,7,10-tetramethyl-1²,6-dioxo-7-aza-1(6,4)-oxazinana-3(2,3)-oxirana-8(1,3)-benzenacyclotetradecaphane-10,12-dien-4-yl *N*-(3-((3-((3-((4-((2-((*S*)-2-cyano-4,4-difluoropyrrolidin-1-yl)-2-oxoethyl)carbamoyl)quinolin-8-yl)oxy)propyl)amino)-3-oxopropyl)disulfaneyl)propanoyl)-*N*-methyl-*L-*alaninate (eFAP-18)
- 2,2',2"-(10-((2S,17S,22S)-22-carboxy-1-(((1⁴S,1⁶S,3²S,3³S,2R,4S,10E, 12E, 14R)-8⁶-chloro-1⁴-hydroxy-8⁵, 14-dimethoxy-3³,2,7,10-tetramethyl-1²,6-dioxo-7-aza-1(6,4)-oxazinana-3(2,3)-oxirana-8(1,3)-benzenacyclotetradecaphane-10,12-dien-4-yl)oxy)-17-((3-((4-((2-((S)-2-cyano-4,4-difluoropyrrolidin-1-yl)-2-oxoethyl)carbamoyl)quinolin-8-yl)oxy)propyl)carbamoyl)-2,3-dimethyl-1,4,11,19-tetraoxo-7,8-dithia-3,12,18-triazadocosan-22-yl)-1,4,7,10-tetraazacyclododecane-1,4,7-triyl)triacetic acid (eFAP-19)
- 2,2',2"-(10-(4-((3-((((1,4-bis((3-((3-((4-((2-((5)-2-cyano-4,4-difluoropyrrolidin-1-yl)-2-oxoethyl)carbamoyl)quinolin-8-yl)oxy)propyl)amino)-3-oxopropyl)thio)-5,6,7,8,9,10-hexahydrocycloocta[*d*]pyridazin-7-yl)oxy)carbonyl)amino)propyl)amino)-1-carboxy-4-oxobutyl)-1,4,7,10-tetraazacyclododecane-1,4,7-triyl)triacetic acid (eFAP-20); and
- radioisotope labeled, preferably ¹¹¹In, ⁶⁸Ga, ¹⁷⁷Lu or ²²⁵Ac radioisotope labeled, eFAP-6, eFAP-8, eFAP-9, eFAP-10, eFAP-11, eFAP-12, eFAP-13, eFAP-14, eFAP-15, eFAP-16, eFAP-19 and eFAP-20.

15. Compound, or a pharmaceutically acceptable salt thereof, according to any of the previous claims for medical use, preferably for use in the diagnosis and/or treatment of tumors.
